# EUROPEAN PATENT SPECIFICATION

(11) **EP 4 406 385 B1**
(45) Date of publication and mention of the grant of the patent: **08.04.2026**
(21) Application number: 22786023.6
(22) Date of filing: 19.09.2022
(51) Int. Cl.: H10K 85/30, C07F 1/08, C07C 49/15, C07C 49/813, C07C 49/92, C07C 255/17, C07D 213/50, C07F 3/06, C07F 5/06, C07F 7/00, C07F 9/94, C07F 15/00, C07F 15/02, H10K 50/17, H10K 101/10

(54) **METAL COMPLEX, SEMICONDUCTOR LAYER COMPRISING A METAL COMPLEX AND ORGANIC ELECTRONIC DEVICE**
METALLKOMPLEX, HALBLEITERSCHICHT MIT EINEM METALLKOMPLEX UND ORGANISCHE ELEKTRONISCHE VORRICHTUNG
COMPLEXE MÉTALLIQUE, COUCHE SEMI-CONDUCTRICE COMPRENANT UN COMPLEXE MÉTALLIQUE ET DISPOSITIF ÉLECTRONIQUE ORGANIQUE

(30) Priority: 20.09.2021 EP 21197750; 20.09.2021 EP 21197667; 20.09.2021 EP 21197757
(43) Date of publication of application: 31.07.2024
(73) Proprietor: Novaled GmbH, 01099 Dresden (DE)
(72) Inventor: UVAROV, Vladimir, 01099 Dresden (DE); WILLMANN, Steffen, 01099 Dresden (DE); HEGGEMANN, Ulrich, 01099 Dresden (DE)
(74) Representative: Michalski Hüttermann & Partner Patentanwälte mbB
(86) International application number: PCT/EP2022/075945
(87) International publication number: WO 2023/041779

(56) References cited:
- WO-A1-03/091227
- WO-A1-2011/117225
- WO-A1-2019/080226
- WO-A1-2021/048044
- DE-A1- 4 330 105
- JP-A- 2010 122 269

## Description

### Technical Field

The present invention relates to a metal complex, a semiconductor layer comprising the metal complex and an organic electronic device comprising at least one metal complex thereof.

### Background Art

Electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode layer, a hole injection layer HIL, a hole transport layer HTL, an emission layer EML, an electron transport layer ETL, and a cathode layer, which are sequentially stacked on a substrate. In this regard, the HIL, the HTL, the EML, and the ETL are thin films formed from organic compounds.

When a voltage is applied to the anode and the cathode, holes injected from the anode move to the EML, via the HIL and HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has low operating voltage, excellent efficiency and/or a long lifetime.

Performance of an organic light emitting diode may be affected by characteristics of the hole injection layer, and among them, may be affected by characteristics of the hole transport compound and the metal complexes which are contained in the hole injection layer.

Performance of an organic light emitting diode may be affected by characteristics of the semiconductor layer, and among them, may be affected by characteristics of metal complexes which are also contained in the semiconductor layer.

There remains a need to improve performance of a metal complex, suitable for use as semiconductor materials, semiconductor layers, as well as electronic devices thereof, in particular to achieve improved operating voltage stability over time through improving the characteristics of the metal complex comprised therein. Iron(III) metal complexes have been disclosed in WO 2011/117225 A1, whereas diketone derivatives have been disclosed in WO 03/091227 A1 and WO 2019/080226 A1.

Further there remains a need to improve performance of a metal complex, suitable for use as semiconductor materials, with good thermal properties and/or solubility in organic solvents.

Further there remains a need to improve performance of electronic devices, especially in view of the long-time stability, by providing semiconductor layers comprising the inventive metal complexes.

### DISCLOSURE

An aspect of the present invention provides a metal complex of formula (I) 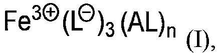 wherein
L has formula (II) 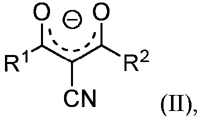
R¹ is selected from substituted C₂ to C₁₂ alkyl, substituted C₆ to C₁₉ aryl, substituted or unsubstituted C₂ to C₂₀ heteroaryl, or substituted or unsubstituted 6-membered heteroaryl;
R² is selected from substituted C₆ to C₁₉ aryl, substituted or unsubstituted C₂ to C₂₀ heteroaryl, substituted or unsubstituted 6-membered heteroaryl;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
L comprises at least two CF₃ groups and/or at least one N atom;
and wherein
AL is an ancillary ligand which coordinates to the metal M;
n is an integer selected from 0 to 2.

A further aspect of the present invention relates to compounds of formula (IIa) as defined in claim 13.

### Definitions

It should be noted that throughout the application and the claims that the metal complex or metal complex of formula (I) is also referred to as compound.

It should be noted that throughout the application and the claims any R¹, R², R³, L and AL etc. always refer independently from each other to the same moieties according to their definition, unless otherwise noted.

In the present specification, when a definition is not otherwise provided, "substituted" refers to a substituted selected from substituted or unsubstituted C₁ to C₁₂ alkyl, partially or fully fluorinated C₁ to C₁₂ alkyl, substituted or unsubstituted C₆ to C₁₉ aryl, and substituted or unsubstituted C₂ to C₂₀ heteroaryl, wherein the substituents are selected from the substituents of the substituted or unsubstituted C₁ to C₁₂ alkyl, substituted or unsubstituted C₆ to C₁₉ aryl, and substituted or unsubstituted C₂ to C₂₀ heteroaryl are selected are independently selected from halogen, Cl, F, CN, partially or perfluorinated C₁ to C₈ alkyl, partially or perfluorinated C₁ to C₈ alkoxy.

In the present specification, "aryl group" and "aromatic rings" refers to a hydrocarbyl group which may be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under "heteroaryl" and "heteroaromatic", it is especially where suitable understood a group derived by formal abstraction of one ring hydrogen from a heterocyclic aromatic ring in a metal complex comprising at least one such ring.

The term "non-heterocycle" is understood to mean a ring or ring-system comprising no hetero-atom as a ring member.

The term "heterocycle" is understood to mean that the heterocycle comprises at least one ring comprising one or more hetero-atoms. A heterocycle comprising more than one ring means that all rings comprising a hetero-atom or at least one ring comprising a hetero atom and at least one ring comprising C-atoms only and no hetero atom. A C₂ heteroaryl group means that an heteroaryl ring comprises two C-Atoms and the other atoms are hetero-atoms.

Under heterocycloalkyl, it is especially where suitable understood a group derived by formal abstraction of one ring hydrogen from a saturated cycloalkyl ring in a compound comprising at least one such ring.

The term "aryl" having at least 9 C-atoms may comprise at least one fused aryl ring. The term "heteroaryl" having at least 9 atoms may comprise at least one fused heteroaryl ring fused with a heteroaryl ring or fused with an aryl ring.

The term "fused aryl rings" or "condensed aryl rings" is understood the way that two aryl rings are considered fused or condensed when they share at least two common sp²-hybridized carbon atoms.

The term "fused ring system" is understood to mean a ring system wherein two or more rings share at least two atoms.

The term "5-, 6- or 7-member ring" is understood to mean a ring comprising 5, 6 or 7 atoms. The atoms may be selected from C and one or more hetero-atoms.

In the present specification, the single bond refers to a direct bond.

In the present specification, when a definition is not otherwise provided, "substituted" refers to one substituted with a H, deuterium, halogen, Cl, F, CN, partially or perfluorinated C₁ to C₈ alkyl, partially or perfluorinated C₁ to C₈ alkoxy.

In the present specification "substituted aryl" refers for example to a C₆ to C₁₉ aryl or C₆ to C₁₈ aryl that is substituted with one or more substituents, wherein the substituent may be substituted with none, one or more substituents, preferably the substituent may be selected from H, deuterium, halogen, Cl, F, CN, partially or perfluorinated C₁ to C₈ alkyl, partially or perfluorinated C₁ to C₈ alkoxy.

Correspondingly, in the present specification "substituted heteroaryl" refers to a substitution with one or more substituents, which themselves may be substituted with one or more substituents, preferably the substituent may be selected from H, deuterium, halogen, Cl, F, CN, partially or perfluorinated C₁ to C₈ alkyl, partially or perfluorinated C₁ to C₈ alkoxy..

In the present specification, when a definition is not otherwise provided, a substituted heteroaryl group with at least 2 C-ring atoms may be substituted with one or more substituents. For example, a substituted C₂ heteroaryl group may have 1 or 2 substituents.

A substituted aryl group with at least 6 ring atoms may be substituted with 1, 2, 3, 4 or 5 substituents.

A substituted heteroaryl group may comprise at least 5 or 6 ring atoms. A substituted heteroaryl group that may comprise at least 5 or 6 ring atoms may be substituted with 1, 2, 3 or 4 substituents, if the heteroaryl group comprises one hetero atom and five C-atoms, or it may be substituted with 1, 2 or 3 substituents, if the heteroaryl group with at least 6 ring atoms comprises two hetero atom and four C-atoms, or may be substituted with 1 or 2 substituents, if the heteroaryl group with at least 6 ring atoms comprises three hetero atoms and three C-atoms, wherein the substituent is bonded to the C-ring atoms only.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, tert-butyl, cyclohexyl.

Specific examples of the alkyl group may be a methyl group, an ethyl group, a propyl group, an iso-propyl group, a butyl group, an iso-butyl group, a sec-butyl group, a tert-butyl group, a pentyl group, a branched pentyl group, a hexyl group, a cyclopropyl group, a cyclobutyl group, a cyclopentyl group, a cyclohexyl group, an adamantly group and the like.

In the present specification, when a definition is not otherwise provided, a "substituted alkyl group" may refer to a linear, branched or cyclic substituted saturated aliphatic hydrocarbyl group. The substituted alkyl group may be a linear, branched or cyclic C₁ to C₁₂ alkyl group. More specifically, the substituted alkyl group may be a linear, branched or cyclic substituted C₁ to C₁₀ alkyl group or a linear, branched or cyclic substituted C₁ to C₆ alkyl group. For example, a linear, branched or cyclic substituted C₁ to C₄ alkyl group includes 1 to 4 carbons in the alkyl chain, and may be selected from methyl, ethyl, propyl, iso-propyl, n-butyl, iso-butyl, sec-butyl, tert-butyl and cyclohexyl. The substituents may be selected from halogen, F, Cl, CN, OCH₃, OCF₃.

The term "hetero" is understood the way that at least one carbon atom, in a structure which may be formed by covalently bound carbon atoms, is replaced by another polyvalent atom. Preferably, the heteroatoms are selected from B, Si, N, P, O, S; further preferred from N, P, O, S and most preferred N.

In the present specification, when a substituent is not named, the substituent may be a H.

The term "charge-neutral" means that the group L is overall electrically neutral.

In the context of the present invention, "different" means that the metal complexes do not have an identical chemical structure.

The term "free of", "does not contain", "does not comprise" does not exclude impurities which may be present in the metal complex prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The term "at least two anode sub-layers" is understood to mean two or more anode sub-layers, for example two or three anode sub-layers.

The terms cathode, cathode layer and cathode electrode are used synonymously.

The term "hole injection layer" is understood to mean a layer which improves charge injection from the anode layer into further layers in the electronic device or from further layers of the electronic device into the anode.

The term "hole transport layer" is understood to mean a layer which transports holes between the hole injection layer and further layers arranged between the hole injection layer and the cathode layer.

The operating voltage U is measured in Volt.

In the context of the present specification the term "essentially non-emissive" or "non-emissive" means that the contribution of the metal complex of formula (I) or the hole injection layer comprising a metal complex of formula (I), to the visible emission spectrum from an electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

In the context of the present invention, the term "sublimation" may refer to a transfer from solid state to gas phase or from liquid state to gas phase.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "LUMO level" is understood to mean the lowest unoccupied molecular orbital and is determined in eV (electron volt).

The term "LUMO level further away from vacuum level" is understood to mean that the absolute value of the LUMO level is higher than the absolute value of the LUMO level of the reference compound.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO level of the matrix compound of the hole injection layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "- "symbol. According to one embodiment of the present invention, the HOMO level of the matrix compound of the hole injection layer may be calculated by quantum mechanical methods.

### Advantageous Effects

Surprisingly, it was found that the electronic device according to the invention solves the problem underlying the present invention by enabling electronic devices, such as organic light-emitting diodes, in various aspects superior over the electronic devices known in the art, in particular with respect to operating voltage stability over time.

The metal complex of formula (I) is non-emissive. In the context of the present specification the term "essentially non-emissive" or "non-emissive" means that the contribution of the metal complex of formula (I) to the visible emission spectrum from an electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

The metal complex of formula (I) can be also named metal complex or metal acetylacetonate complex as well as iron complex or iron acetylacetonate complex.

According to one embodiment, the metal complex of formula (I) may have a molecular weight Mw of ≥ 287 g/mol and ≤ 2000 g/mol, preferably a molecular weight Mw of ≥ 400 g/mol and ≤ 2000 g/mol, further preferred a molecular weight Mw of ≥ 580 g/mol and ≤ 2000 g/mol, in addition preferred a molecular weight Mw of ≥ 1000 g/mol and ≤ 2000 g/mol, in addition preferred a molecular weight Mw of ≥ 1000 g/mol and ≤ 1500 g/mol.

### Ligand L of formula (II)

The term "L" is preferably represented by formula (II) 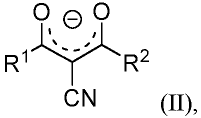
R¹ is selected from substituted C₂ to C₁₂ alkyl, substituted C₆ to C₁₉ aryl, substituted or unsubstituted C₂ to C₂₀ heteroaryl, or substituted or unsubstituted 6-membered heteroaryl;
R² is selected from substituted C₆ to C₁₉ aryl, substituted or unsubstituted C₂ to C₂₀ heteroaryl, substituted or unsubstituted 6-membered heteroaryl;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to Cs alkyl, partially or perfluorinated C₁ to Cs alkoxy;
wherein
at least one of R¹ and R² or the group of R¹ and R² comprises at least two CF₃ groups and/or at least one N atom;

According to one embodiment of the application, R¹ comprises at least two CF₃ groups and/or at least one N atom.

According to one embodiment of the application, R¹ is selected from substituted C₆ to C₁₉ aryl, substituted or unsubstituted C₂ to C₂₀ heteroaryl, or substituted or unsubstituted 6-membered heteroaryl.

According to one embodiment of the application, R¹ comprises two CF₃ groups and at least further substituent selected from CN, CF₃, C₂F₅, C₃F₇ or C₄F₉.

According to one embodiment of the application, R¹ comprises two CF₃ groups and is otherwise unsubstituted.

According to one embodiment of the application, R¹ comprises an aromatic six-membered ring, which either can be aryl or heteroaryl.

According to one embodiment of the application, R¹ comprises an aromatic six-membered ring with two CF₃ groups being in meta-position to the 1,3-diketo moiety.

According to one embodiment of the application R¹ comprises an aromatic six-membered ring which is unsubstituted in ortho-position to the 1,3-diketo moiety.

According to one embodiment of the application, R¹ comprises an aromatic six-membered ring with at least one N atom.

According to one embodiment of the application, R¹ comprises an aromatic six-membered ring with at least one N atom, whereby at least one N atom is in para-position to the 1,3-diketo moiety.

According to one embodiment of the application R¹ and R² are identical.

According to one embodiment of the application, R² comprises an unsubstituted or substituted aromatic six-membered ring, which either can be aryl or heteroaryl, an unsubstituted or substituted aromatic five-membered ring, which comprises at least one of O, N and S, or C₂F₅, C₃F₇ or C₄F₉.

According to one embodiment of the application R¹ and/or R² are selected from the following moieties D1 to D70 (in consideration of the above provisos for L): 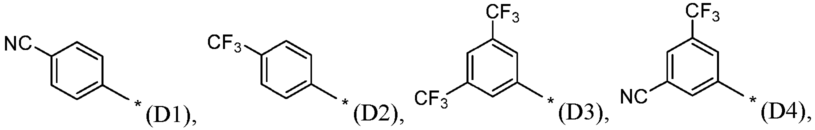 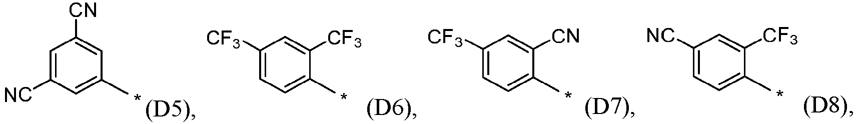 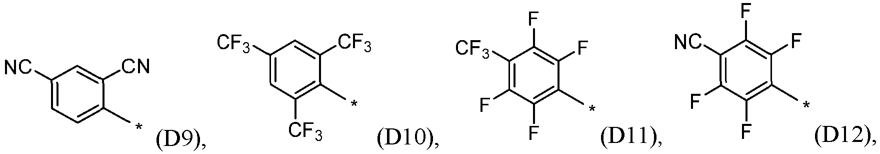 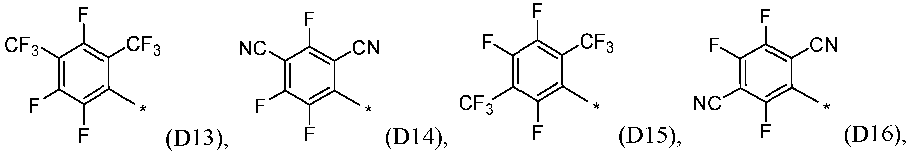 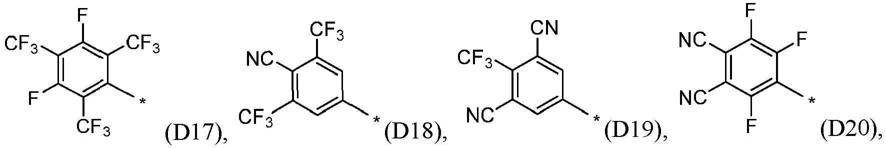 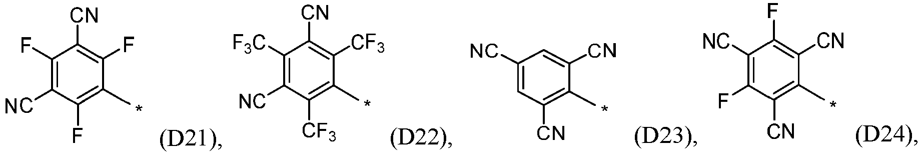 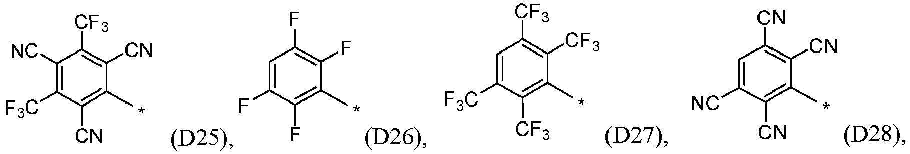 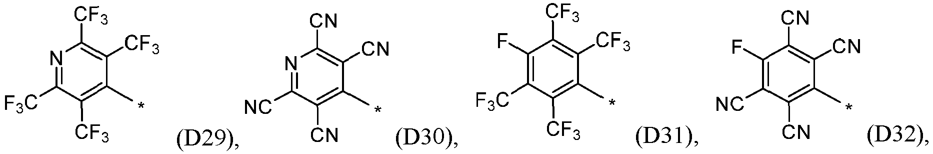 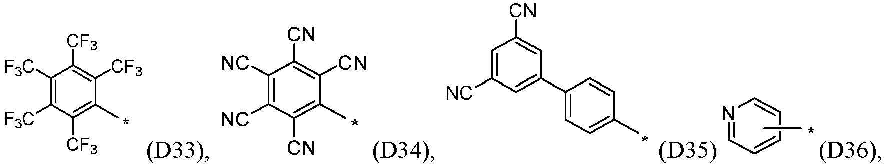 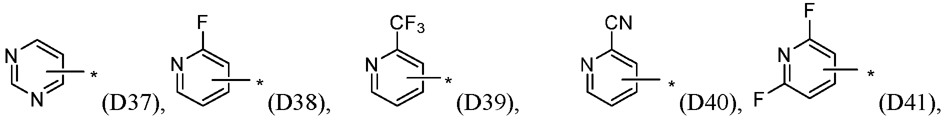 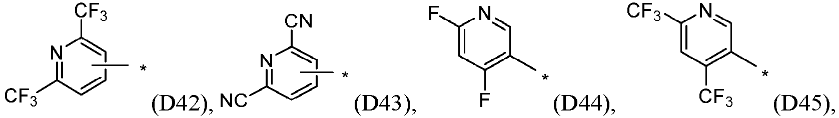 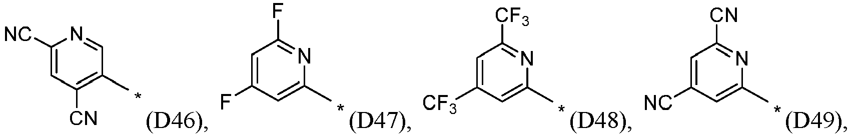 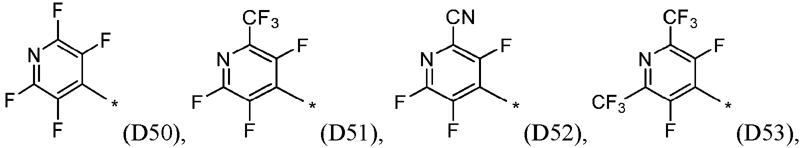 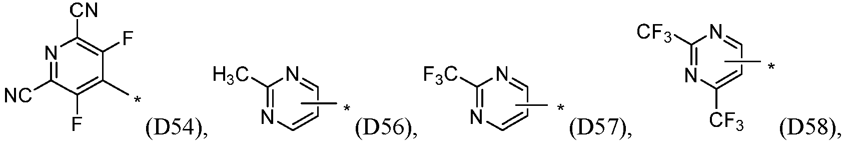 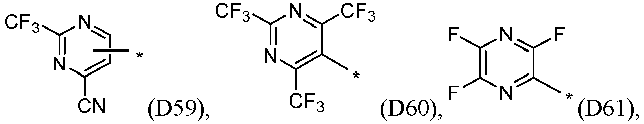 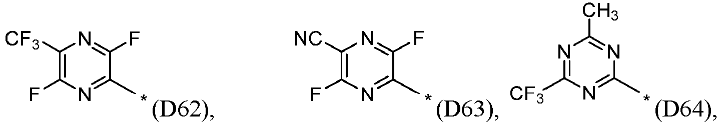 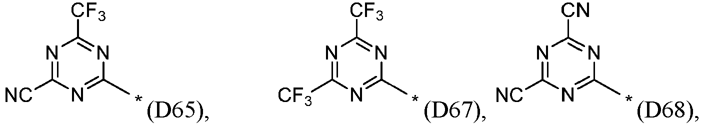 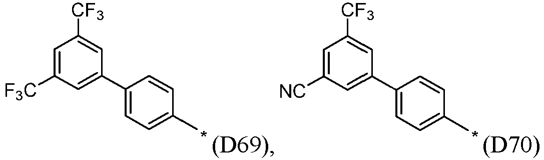 wherein the "*" denotes the binding position.

According to one embodiment of the application, the ligand L is selected from one of the following structures L1 to L54

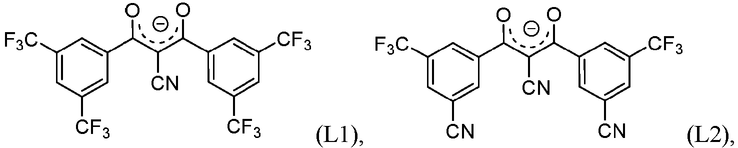

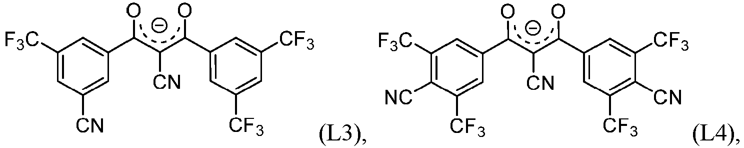

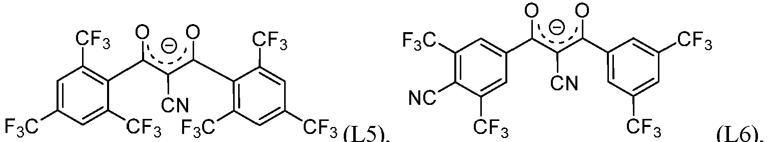

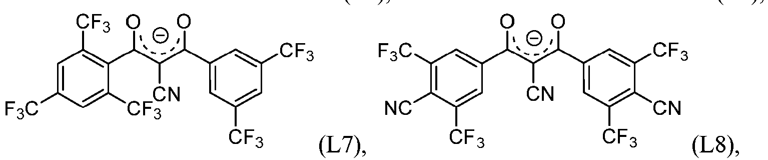

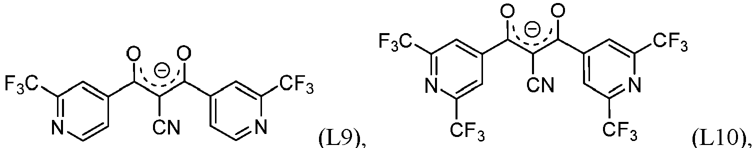

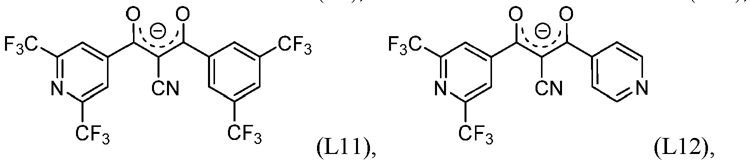

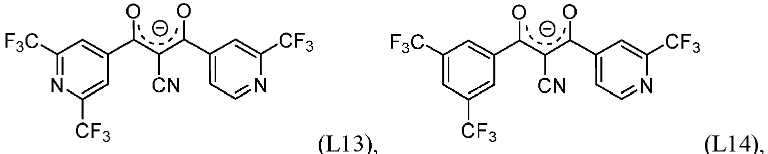

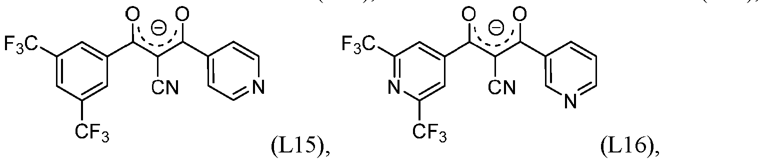

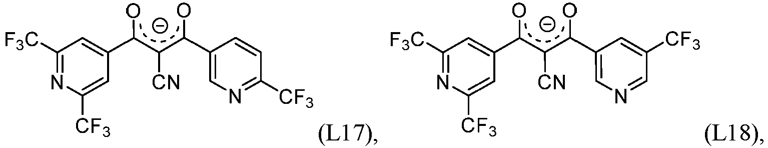

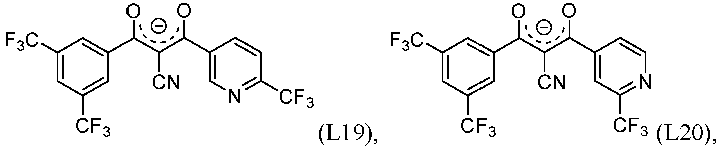

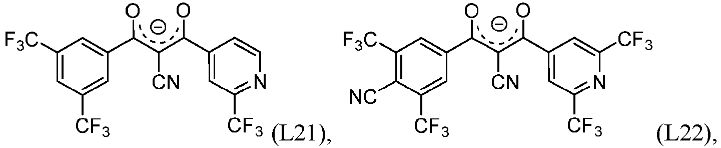

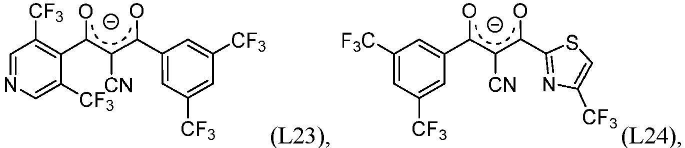

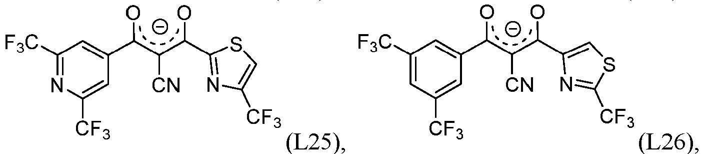

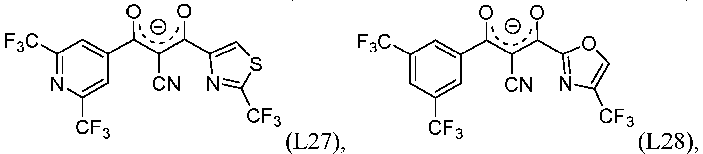

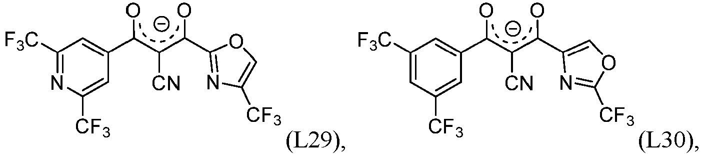

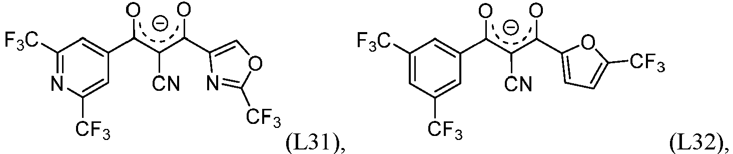

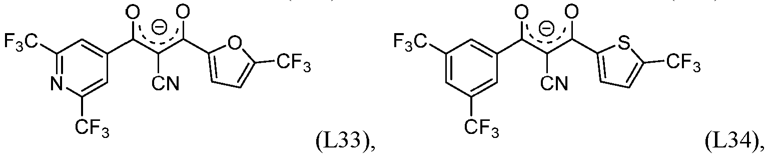

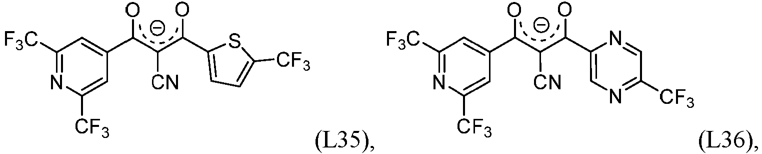

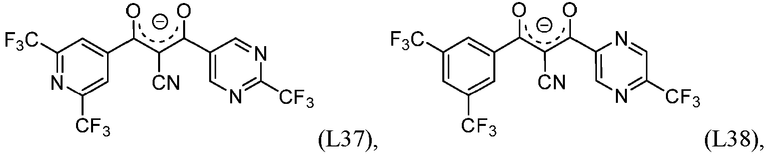

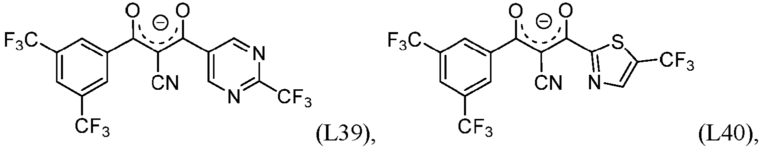

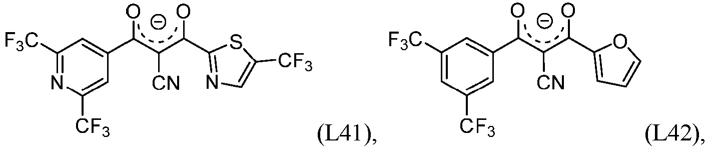

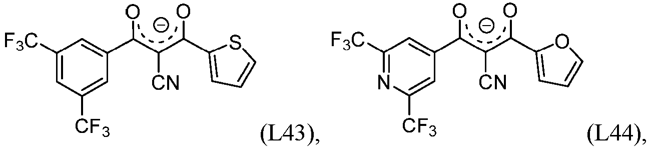

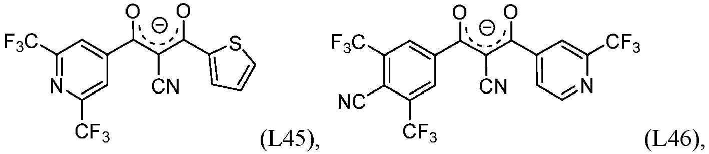

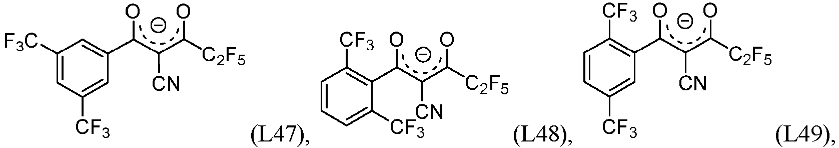

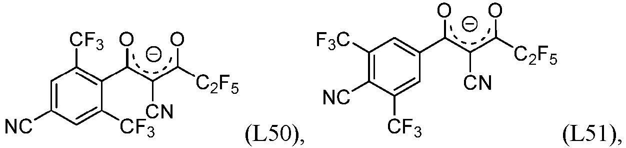

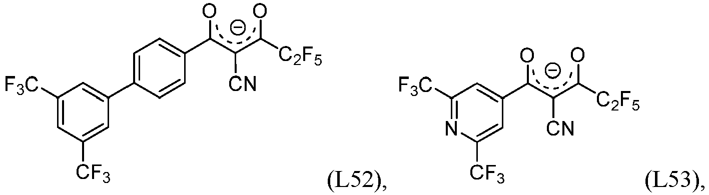

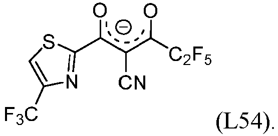

According to one embodiment of the application, the ligand L is selected from one of L1 to L46, preferably L1 to L22.

According to one embodiment of the application AL is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (III); 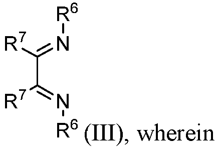 wherein
- R⁶ and R⁷: are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

### Semiconductor material

According to another aspect, a semiconductor material comprises at least one compound of Formula (I) according to the present invention.

According to one embodiment, wherein the semiconductor material comprises in addition at least one covalent matrix compound or at least one substantially covalent matrix compound.

According to another aspect, wherein a semiconductor material comprises at least one compound of Formula (I) according to the present invention and in addition at least one covalent matrix compound or at least one substantially covalent matrix compound.

### Substantially covalent matrix compound/ covalent matrix compound

The substantially covalent matrix compound, also named matrix compound, may be an organic aromatic matrix compounds, which comprises organic aromatic covalent bonded carbon atoms. The substantially covalent matrix compound may be an organic compound, consisting substantially from covalently bound C, H, O, N, S, which may optionally comprise also covalently bound B, P or Si. The substantially covalent matrix compound may be an organic aromatic covalent bonded compound, which is free of metal atoms, and the majority of its skeletal atoms may be selected from C, O, S, N and preferably from C, O and N, wherein the majority of atoms are C-atoms. Alternatively, the covalent matrix compound is free of metal atoms and majority of its skeletal atoms may be selected from C and N, preferably the covalent matrix compound is free of metal atoms and majority of its skeletal atoms may be selected from C and the minority of its skeletal atoms may be N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment of the present invention, the substantially covalent matrix compound may be free of alkoxy groups.

Preferably, the substantially covalent matrix compound comprises at least one arylamine moiety, alternatively a diarylamine moiety, alternatively a triarylamine moiety.

Preferably, the substantially covalent matrix compound is free of TPD or NPB.

### Compound of formula (IV) or a compound of formula (V)

According to the present invention, the substantially covalent matrix compound or covalent matrix compound, also referred to as matrix compound herein, may comprises at least one arylamine compound, diarylamine compound, triarylamine compound, a compound of formula (IV) or a compound of formula (V): 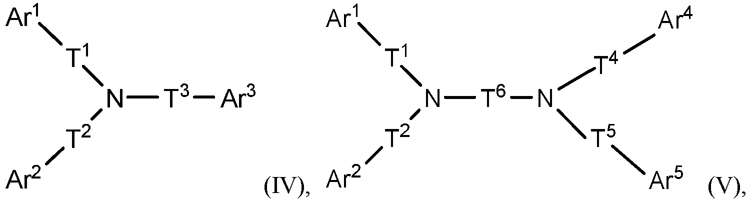 wherein:
- T¹, T², T³, T⁴ and T⁵: are independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment of the electronic device, wherein the substantially covalent matrix compound comprises a compound of formula (IV) or formula (V): 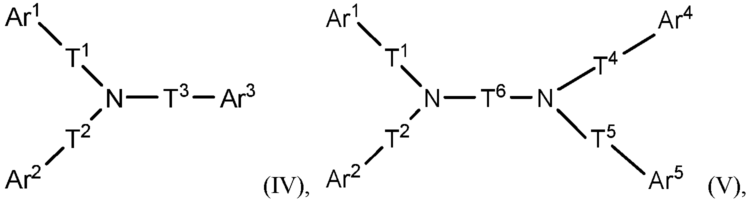 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein the substituents of Ar¹, Ar², Ar³, Ar⁴ and Ar⁵ are selected the same or different from the group comprising H, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, C₆ to C₁₈ aryl, C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl, C₃ to C₁₈ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of **H,** straight-chain alkyl having 1 to 4 carbon atoms, branched alkyl having 1 to 4 carbon atoms, cyclic alkyl having 3 to 4 carbon atoms and/or phenyl.
Thereby, the compound of formula (IV) or (V) may have a rate onset temperature suitable for mass production.

According to an embodiment of the electronic device, wherein the substantially covalent matrix compound comprises a compound of formula (IV) or formula (V): 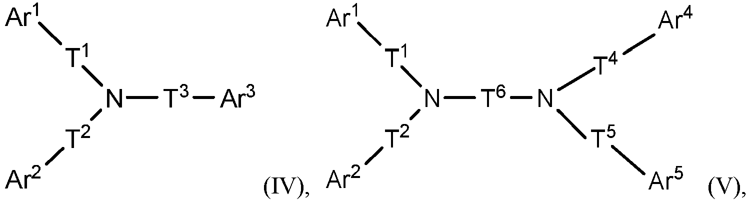 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment of the electronic device, wherein the substantially covalent matrix compound comprises a compound of formula (IV) or formula (V): 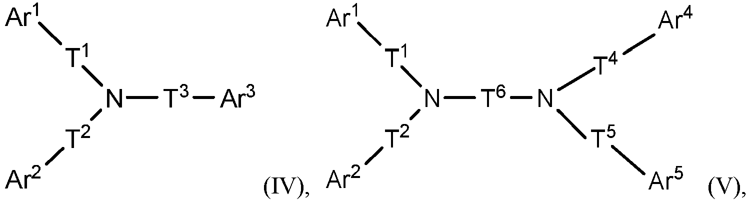 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IV) or (V) may have a rate onset temperature suitable for mass production.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from B1 to B16: 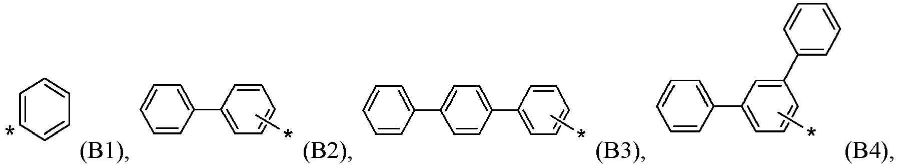 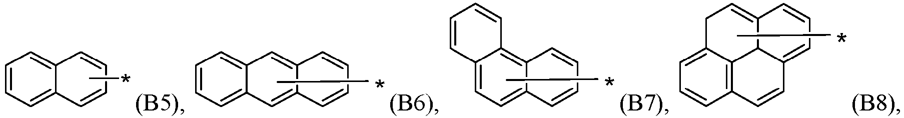 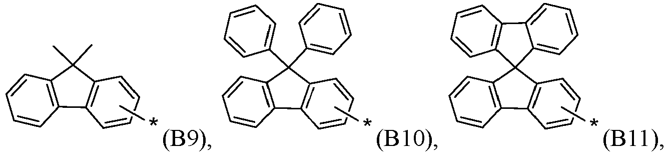 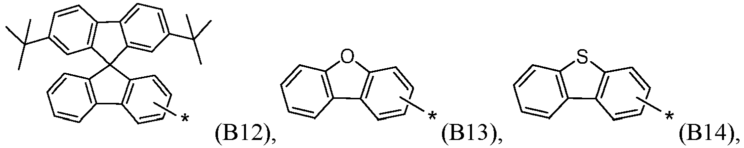 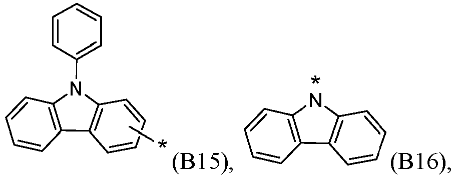 wherein the asterix "*" denotes the binding position.

According to an embodiment, wherein Ar¹, Ar², Ar³, Ar⁴ and Ar⁵ may be independently selected from B1 to B15; alternatively selected from B1 to B10 and B13 to B15.

According to an embodiment, wherein Ar¹, Ar², Ar³, Ar⁴ and Ar⁵ may be independently selected from the group consisting of B1, B2, B5, B7, B9, B10, B13 to B16.

The rate onset temperature may be in a range particularly suited to mass production, when Ar¹, Ar², Ar³, Ar⁴ and Ar⁵ are selected in this range.

The "matrix compound of formula (IV) or formula (V) " may be also referred to as "hole transport compound".

According to one embodiment the compound of formula (IV) or formula (V) may comprises at least ≥ 1 to ≤ 6 substituted or unsubstituted aromatic fused ring systems comprising heteroaromatic rings.

According to one embodiment the compound of formula (IV) or formula (V) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the compound of formula (IV) or formula (V) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings.

According to one embodiment the compound of formula (IV) or formula (V) may comprises at least ≥ 1 to ≤ 3 or 2 substituted or unsubstituted unsaturated 5- to 7-member ring of a heterocycle.

According to one embodiment the compound of formula (IV) or formula (V) may comprises at least ≥ 1 to ≤ 3 or 2 substituted or unsubstituted unsaturated 7-member ring of a heterocycle.

According to one embodiment substituted or unsubstituted aromatic fused ring systems of the compound of formula (IV) or formula (V) may comprises at least ≥ 1 to ≤ 3 or 2 substituted or unsubstituted unsaturated 5- to 7-member ring of a heterocycle.

According to one embodiment the substituted or unsubstituted aromatic fused ring systems of the matrix compound of formula (IV) or formula (V) may comprises at least ≥ 1 to ≤ 3 or 2 substituted or unsubstituted unsaturated 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises substituted or unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ **2 to** ≤ **5** substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises substituted or unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises at least ≥ 1 to ≤ 3 or 2 substituted or unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises at least ≥ 1 to ≤ 3 or 2 substituted or unsubstituted unsaturated 5- to 7-member ring of a heterocycle.

According to one embodiment the compound of formula (IV) or formula (V) may comprises:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

**According** to one embodiment the compound of formula (IV) or formula (V) may comprises at least at least ≥ 1 to ≤ 6, preferably ≥ 2 to ≤ 5, or further preferred 3 or 4 of the substituted or unsubstituted aromatic fused ring systems with:
- at least one unsaturated 5-member ring, and/or
- at least one unsaturated 6-member ring, and/or
- at least one unsaturated 7-member ring; wherein preferably at least one unsaturated 5- and/or at least one unsaturated 7-member ring comprises at least 1 to 3, preferably 1 hetero-atom.

According to one embodiment the compound of formula (IV) or formula (V) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system comprises at least ≥ 1 to ≤ 3 or 2 substituted or unsubstituted unsaturated 5- to 7-member ring of a heterocycle.

According to one embodiment the compound of formula (IV) or formula (V) may comprises:
- at least ≥ 6 to ≤ 12, preferably ≥ 7 to ≤ 11, further preferred ≥ 8 to ≤ 10 or 9 aromatic rings; and/or
- at least ≥ 4 to ≤ 11, preferably ≥ 5 to ≤ 10, further preferred ≥ 6 to ≤ 9 or in addition preferred 7 or 8 non-hetero aromatic rings, preferably the non-hetero aromatic rings are aromatic C₆ rings; and/or
- at least ≥ 1 to ≤ 4, preferably 2 or 3 aromatic 5-member-rings, preferably hetero aromatic 5-member-rings; and/or
- at least 1 or 2 unsaturated 5- to 7-member-ring of a heterocycle, preferably at least 1 or 2 unsaturated 7-member-ring of a heterocycle;
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and/or
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and
      the hole transport compound or the hole transport compound according to formula (I) comprises at least ≥ 1 to ≤ 4, preferably 2 or 3 aromatic 5-member-rings, preferably hetero aromatic 5-member-rings, and/or
      the hole transport compound or the hole transport compound according to formula (I) comprises at least 1 or 2 unsaturated 5- to 7-member-ring of a heterocycle, preferably at least 1 or 2 unsaturated 7-member-ring of a heterocycle.

According to one embodiment the compound of formula (IV) or formula (V) may comprises a hetero-atom, which may be selected from the group comprising O, S, N, B or P, preferably the hetero-atom may be selected from the group comprising O, S or N.

According to one embodiment the matrix compound of formula (IV) or formula (V) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
   wherein the substituted or unsubstituted aromatic fused ring system optional comprises at least ≥ 1 to ≤ 3 or 2 substituted or unsubstituted unsaturated 5- to 7-member ring of a heterocycle; and wherein the substituted or unsubstituted aromatic fused ring system comprises a hetero-atom, which may be selected from the group comprising O, S, N, B, P or Si, preferably the hetero-atom may be selected from the group comprising O, S or N.

According to one embodiment the compound of formula (IV) or formula (V) may be free of hetero-atoms which are not part of an aromatic ring and/or part of an unsaturated 7-member-ring, preferably the hole transport compound or the hole transport compound according to formula (I) may be free on N-atoms except N-atoms which are part of an aromatic ring or are part of an unsaturated 7-member-ring.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the present invention, wherein the compound of formula (IV) or formula (V) are selected from K1 to K16:

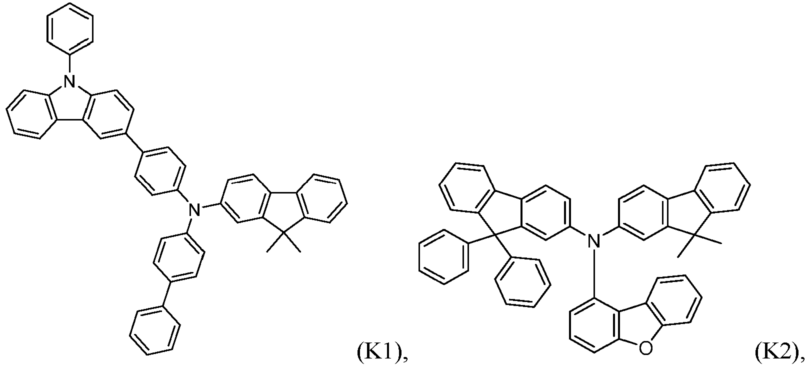

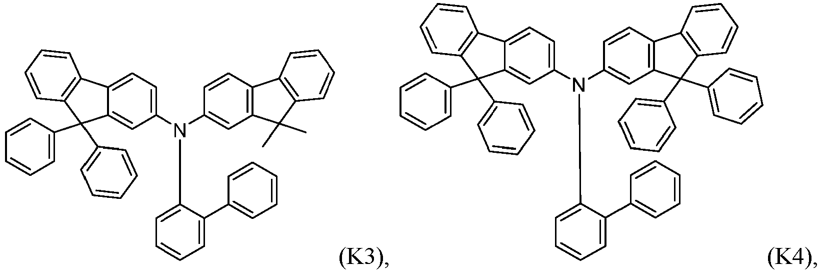

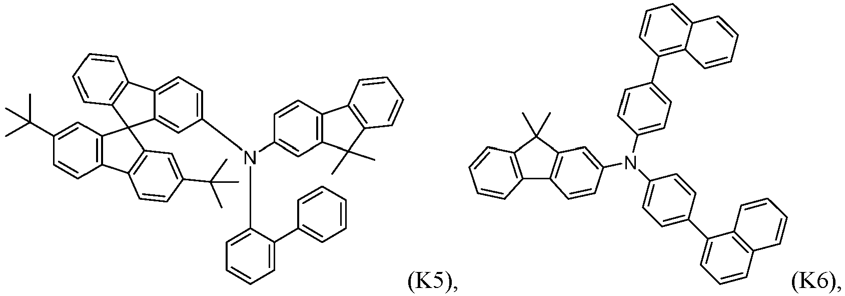

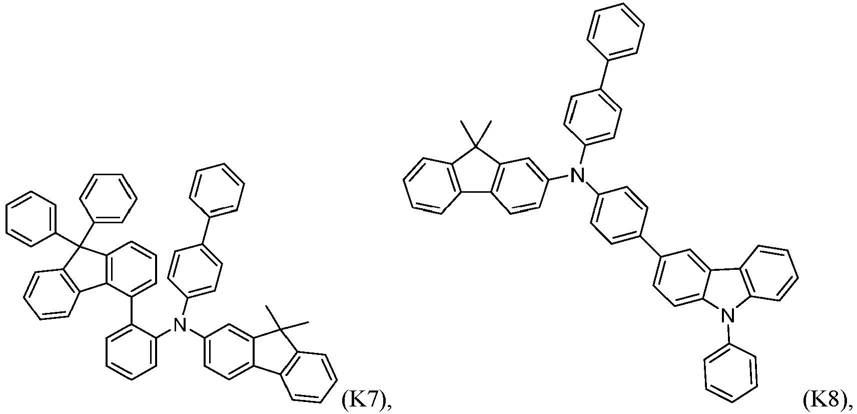

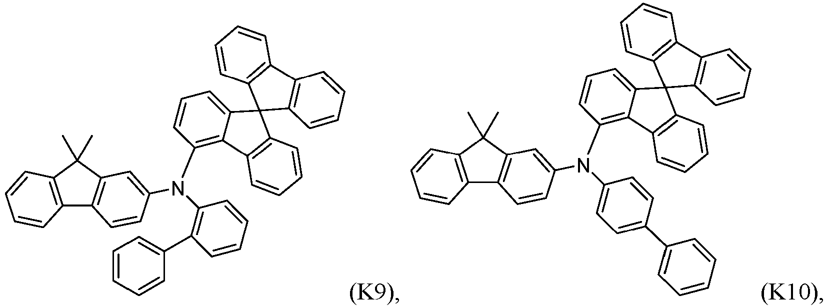

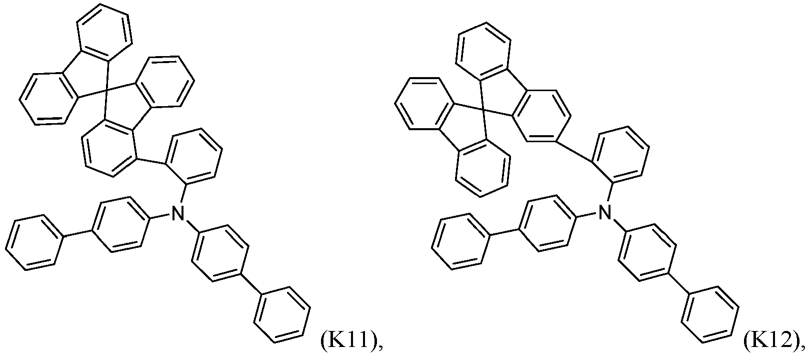

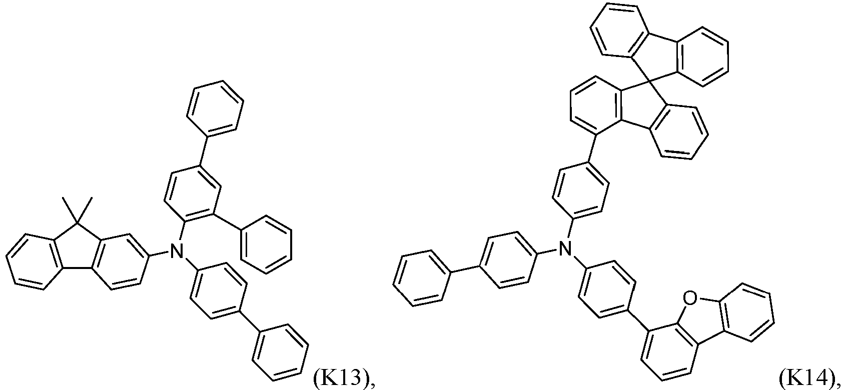

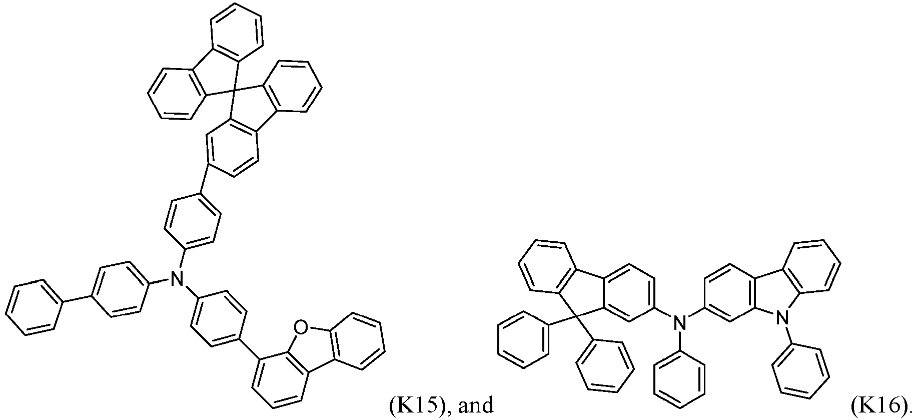

The substantially covalent matrix compound may be free of HTM014, HTM081, HTM163, HTM222, EL-301, HTM226, HTM355, HTM133, HTM334, HTM604 and EL-22T. The abbreviations denote the manufacturers' names, for example, of Merck or Lumtec.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the hole transport compound that is used to form the HIL, and the desired structure and thermal properties of the HIL. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the hole transport compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of a metal complex according to formula (I).

The thickness of the HIL may be in the range from about 1 nm to about 15 nm, and for example, from about 2 nm to about 15 nm, alternatively about 2 nm to about 12 nm.

When the thickness of the HIL is within this range, the HIL may have excellent hole injecting characteristics, without a substantial penalty in driving voltage.

According to one embodiment of the present invention, the hole injection layer may comprise:
- at least about ≥ 0.5 wt.-% to about ≤ 30 wt.-%, preferably about ≥ 0.5 wt.-% to about ≤ 20 wt.- %, and more preferred about ≥ 1 wt.-% to about ≤ 15 wt.-% of a metal complex according to formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the a metal complex according to formula (I) is lower than the wt.-% of the substantially covalent matrix compound; wherein the weight-% of the components are based on the total weight of the hole injection layer.

Preferably, the hole injection layer may be free of ionic liquids, metal phthalocyanine, CuPc, HAT-CN, Pyrazino[2,3-f][1,10]phenanthroline-2,3-dicarbonitrile, F₄TCNQ, metal fluoride and/or metal oxides, wherein the metal in the metal oxide is selected from Re and/or Mo. Thereby, the hole injection layer may be deposited under conditions suitable for mass production.

According to an embodiment of the electronic device, wherein the hole injection layer is non-emissive.

It is to be understood that the hole injection layer is not part of the anode layer.

### Hole transport layer / Hole injection layer

According to a further embodiment the organic electronic device further comprises a hole transport layer, wherein the hole transport layer is arranged between the hole injection layer and the at least one emission layer.

In one embodiment of the present invention, the hole injection layer comprises a substantially covalent matrix compound.

In one embodiment of the present invention, the hole transport layer comprises a substantially covalent matrix compound.

In one embodiment of the present invention, the hole transport layer comprises a substantially covalent matrix compound, wherein the substantially covalent matrix compound in the hole injection layer and hole transport layer are selected the same.

Preferably, the matrix compound of the hole injection layer is free of metals and/or ionic bonds.

### Further layers

In accordance with the invention, the electronic device may comprise, besides the layers already mentioned above, further layers. Exemplary embodiments of respective layers are described in the following:

### Substrate

The substrate may be any substrate that is commonly used in manufacturing of electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a transistor backplane. Preferably, the substrate is a silicon substrate or transistor backplane.

### Anode layer

The anode layer, also named anode electrode, may be formed by depositing or sputtering a material that is used to form the anode layer. The material used to form the anode layer may be a high work-function material, so as to facilitate hole injection. The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode layer. The anode layer may also be formed using metals, typically silver (Ag), gold (Au), or metal alloys.

The anode layer may comprise two or more anode sub-layers.

According to one embodiment, the anode layer comprises a first anode sub-layer and a second anode sub-layer, wherein the first anode sub-layer is arranged closer to the substrate and the second anode sub-layer is arranged closer to the cathode layer.

According to one embodiment, the anode layer may comprise a first anode sub-layer comprising or consisting of Ag or Au and a second anode-sub-layer comprising or consisting of transparent conductive oxide.

According to one embodiment, the anode layer comprises a first anode sub-layer, a second anode sub-layer and a third anode sub-layer, wherein the first anode sub-layer is arranged closer to the substrate and the second anode sub-layer is arranged closer to the cathode layer, and the third anode sub-layer is arranged between the substrate and the first anode sub-layer.

According to one embodiment, the anode layer may comprise a first anode sub-layer comprising or consisting of Ag or Au, a second anode-sub-layer comprising or consisting of transparent conductive oxide and optionally a third anode sub-layer comprising or consisting of transparent conductive oxide. Preferably the first anode sub-layer may comprise or consists of Ag, the second anode-sublayer may comprise or consists of ITO or IZO and the third anode sub-layer may comprises or consists of ITO or IZO.

Preferably the first anode sub-layer may comprise or consists of Ag, the second anode-sublayer may comprise or consists of ITO and the third anode sub-layer may comprise or consist of ITO.

Preferably, the transparent conductive oxide in the second and third anode sub-layer may be selected the same.

According to one embodiment, the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm.

### Hole transport layer

According to one embodiment of the electronic device, wherein the electronic device further comprises a hole transport layer, wherein the hole transport layer is arranged between the hole injection layer and the at least one first emission layer.

The hole transport layer may comprise a substantially covalent matrix compound. According to one embodiment the substantially covalent matrix compound of the hole transport layer may be selected from at least one organic compound. The substantially covalent matrix may consist substantially from covalently bound C, H, O, N, S, which optionally comprise in addition covalently bound B, P, As and/or Se.

According to one embodiment of the electronic device, the hole transport layer comprises a substantially covalent matrix compound, wherein the substantially covalent matrix compound of the hole transport layer may be selected from organic compounds consisting substantially from covalently bound C, H, O, N, S, which optionally comprise in addition covalently bound B, P, As and/or Se.

According to one embodiment, the substantially covalent matrix compound of the hole transport layer may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound of the hole injection layer and the substantially covalent matrix compound of the hole transport layer are selected the same.

According to one embodiment of the electronic device, wherein the hole transport layer of the electronic device comprises a substantially covalent matrix compound, preferably the substantially covalent matrix compound in the hole injection layer and hole transport layer are selected the same.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the hole transport compound that is used to form the HTL.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 200 nm, further about 100 nm to about 180 nm, further about 110 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer may be achieved. The triplet control layer may be selected from triarylamine compounds with a triplet level above the triplet level of the phosphorescent emitter in the adjacent emission layer.

The thickness of the electron blocking layer may be selected between 2 and 20 nm.

### Photoactive layer (PAL)

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the a metal complex according to formula (I). The photoactive layer may be a light-emitting layer or a light-absorbing layer.

### Emission layer (EML)

The at least one first emission layer (EML), also referred to as first emission layer may be formed on the HTL or EBL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to the present invention it is preferred that the electronic device comprises one emission layer that is named "first emission layer". However, the electronic device optionally comprises two emission layers, wherein the first layer is named first emission layer and second layer is named second emission layer.

It may be provided that the at least one emission layer also referred to as first emission layer is free of the matrix compound of the hole injection layer.

It may be provided that the at least one emission layer does not comprise the a metal complex according to formula (I).

The at least one emission layer (EML) may be formed of a combination of a host and an emitter dopant. Example of the host are Alq3, 4,4'-N,N'-dicarbazole-biphenyl (HTC-10), poly(n-vinyl carbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters; however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)₂Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the at least one emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent emitter dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form an HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitate injection of electrons from the cathode, may be formed on the ETL, preferably directly on the electron transport layer. Examples of materials for forming the EIL include lithium 8-hydroxyquinolinolate (LiQ), LiF, NaCl, CsF, Li₂O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

The present invention furthermore relates to a compound of formula (IIa) 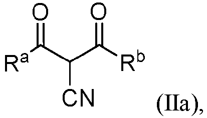 whereby
R^{a} is selected from substituted C₂ to C₁₂ alkyl, substituted C₆ to C₁₉ aryl, substituted or unsubstituted C₂ to C₂₀ heteroaryl, or substituted or unsubstituted 6-membered heteroaryl;
R^{b} is selected from substituted C₂ to C₂₀ heteroaryl or substituted 6-membered heteroaryl;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
the compound of formula (IIa) comprises at least two CF₃ groups and/or at least one N atom.

These compounds can be used as ligands for metal complexes which may be used in organic electronic devices.

According to one embodiment, R^{b} is selected from the formulae D38 to D68 as described above.

According to one embodiment R^{a} is selected from substituted C₂ to C₁₂ alkyl and substituted C₆ to C₁₉ aryl and R^{b} is selected from formulae D39, D42, D45, D48, D56, D57, D58, D67.

According to one embodiment, R^{a} and R^{b} are independently selected from the formulae D39, D42, D45, D48, D56, D57, D58, D67 as described above.

### Organic electronic device

According to another aspect of the present invention an organic electronic device is provided, wherein the organic electronic device comprises a compound of Formula (I).

According to one embodiment of the present invention, the organic electronic device is selected from the group comprising a light emitting device, thin film transistor, a battery, a display device or a photovoltaic cell, and preferably a light emitting device, preferably the electronic device is part of a display device or lighting device.

According to one embodiment the organic electronic device comprises a semiconductor material, wherein at least one semiconductor material comprises a metal complex of Formula (I).

According to one embodiment the organic electronic device comprises a metal complex according to Formula (I) of the present invention is a light emitting device, a thin film transistor, a battery, a display device or a photovoltaic device, and preferably a light emitting device, preferably the electronic device is part of a display device or lighting device.

According to one embodiment the organic electronic device comprises a semiconductor material, wherein at least one semiconductor material comprises a metal complex of Formula (I), and wherein the organic electronic device is a light emitting device, a thin film transistor, a battery, a display device or a photovoltaic device, and preferably a light emitting device, preferably the electronic device is part of a display device or lighting device.

According to one embodiment the organic electronic device comprises a first electrode layer, a second electrode layer, at least one photoactive layer and a semiconductor layer, wherein the semiconductor layer is arranged between the first electrode layer and the at least one photoactive layer, the semiconductor layer comprises a metal complex of formula (I) 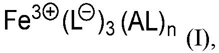 wherein
L has formula (II) 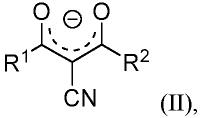
R¹ is selected from substituted C₂ to C₁₂ alkyl, substituted C₆ to C₁₉ aryl, substituted or unsubstituted C₂ to C₂₀ heteroaryl, or substituted or unsubstituted 6-membered heteroaryl;
R² is selected from substituted C₆ to C₁₉ aryl, substituted or unsubstituted C₂ to C₂₀ heteroaryl, substituted or unsubstituted 6-membered heteroaryl;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
L comprises at least two CF₃ groups and/or at least one N atom;
and wherein
AL is an ancillary ligand which coordinates to the metal M;
n is an integer selected from 0 to 2.

Further embodiments of the metal complex of formula (I) are described throughout the present description to which references are fully relayed too.

### Method of manufacturing

According to another aspect, there is provided a method of manufacturing an electronic device, the method using:
- at least one deposition source, preferably two deposition sources and more preferred at least three deposition sources.

The methods for deposition that may be suitable comprise:
- deposition via vacuum thermal evaporation;
- deposition via solution processing, preferably the processing may be selected from spin-coating, printing, casting; and/or
- slot-die coating.

According to various embodiments, there is provided a method using:
- a first deposition source to release the matrix compound, and
- a second deposition source to release the a metal complex according to formula (I), also named metal complex.

The method comprising the steps of forming the hole injection layer; whereby for an electronic device:
- the hole injection layer is formed by releasing the matrix compound according to the invention from the first deposition source and the a metal complex according to formula (I), also named metal complex, from the second deposition source.

### Ink formulation

According to another aspect of the present invention an ink formulation is provided, wherein the ink formulation comprises at least one compound of Formula (I).

According to one embodiment the ink formulation comprises a solvent. The solvent of the ink formulation may be selected from anisole and/or benzonitrile, preferably it is a mixture of anisole and benzonitrile.

According to one embodiment the mixture of anisole and benzonitrile is of 5 : 1 to 1 : 5, 4 : 1 to 1 : 4, 3 : 1 to 1 : 3, 2 : 1 to 1 : 2 or 1:1; preferred is a mixture of anisole and benzonitrile of 5:1.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiment according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
- FIG. 1: is a schematic sectional view of an organic electronic device, according to an exemplary embodiment of the present invention;
- FIG. 2: is a schematic sectional view of an organic electronic device, according to an exemplary embodiment of the present invention;
- FIG. 3: is a schematic sectional view of an organic electronic device , according to an exemplary embodiment of the present invention;
- FIG. 4: is a schematic sectional view of an OLED, according to an exemplary embodiment of the present invention.
- FIG. 5: is a schematic sectional view of an OLED, according to an exemplary embodiment of the present invention.
- FIG. 6: is a schematic sectional view of an OLED, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

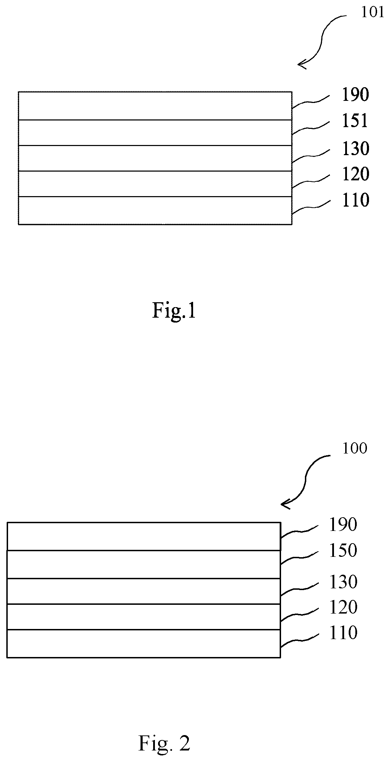

FIG. 1 is a schematic sectional view of an organic electronic device 100, according to an exemplary embodiment of the present invention. The organic electronic device 100 includes an anode layer 120 and an organic semiconductor layer 131 which may comprise a metal complex of formula (I). The organic semiconductor layer 131 is disposed on the anode layer 120. Onto the organic semiconductor layer 131 a cathode layer 190 are disposed.

FIG. 2 is a schematic sectional view of an organic electronic device 100, according to an exemplary embodiment of the present invention. The organic electronic device 100 includes a substrate 110, an anode layer 120 and a hole injection layer (HIL) 130 which may comprise a metal complex of formula (I). The HIL 130 is disposed on the anode layer 120. Onto the HIL 130, a photoactive layer (PAL) 170 and a cathode layer 190 are disposed.

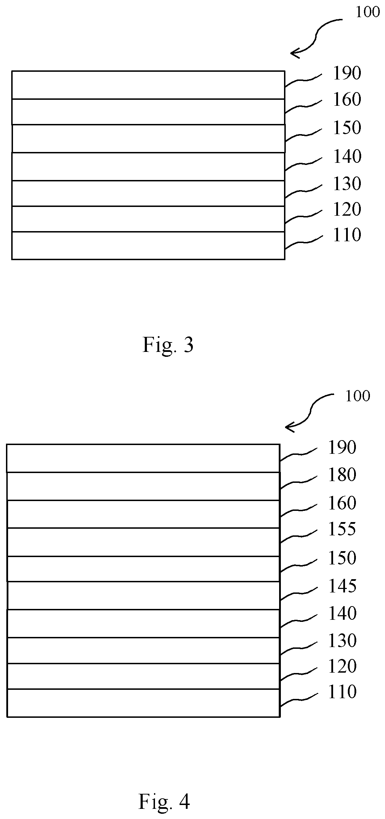

FIG. 3 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a metal complex of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

FIG. 4 is a schematic sectional view of an OLED 100, according to another exemplary embodiment of the present invention. Fig. 4 differs from Fig. 3 in that the OLED 100 of Fig. 4 comprises an electron blocking layer (EBL) 145 and a hole blocking layer (HBL) 155.

Referring to Fig. 4, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

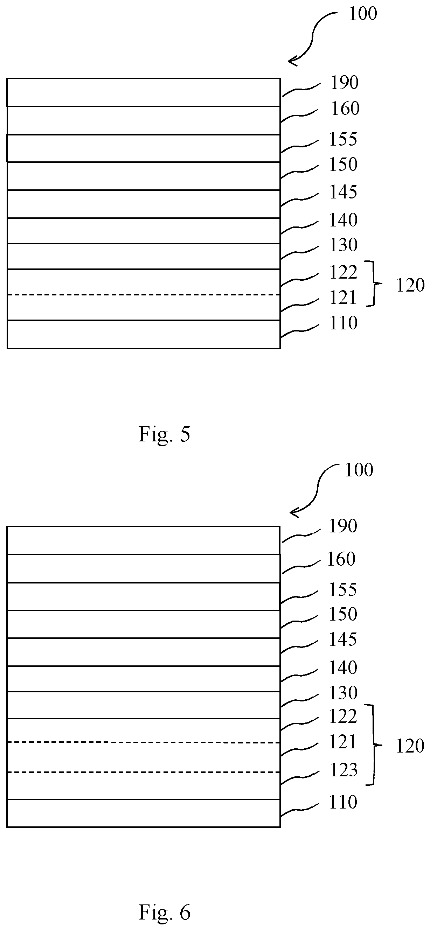

FIG. 5 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, an hole transport layer (HTL) 140, a first emission layer (EML) 150, a hole blocking layer (HBL) 155, an electron transport layer (ETL) 160, and a cathode layer 190 are disposed. The hole injection layer 130 may comprise a metal complex of formula (I).

FIG. 6 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, a hole transport layer (HTL) 140, an electron blocking layer (EBL) 145, a first emission layer (EML) 150, a hole blocking layer (HBL) 155, an electron transport layer (ETL) 160, an electron injection layer (EIL) 180 and a cathode layer 190 are disposed. The hole injection layer 130 may comprise a metal complex of formula (I).

While not shown in Fig. 1 to Fig. 6, a capping and/or sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Calculated LUMO of the metal complex of formula (I)

The HOMO and LUMO are calculated with the program package ORCA V5.0.3 (Max Planck Institute fuer Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The optimized geometries and the HOMO and LUMO energy levels of the molecular structures are determined by applying the hybrid functional (Gaussian)-B3LYP with a ZORA-Def2-TZVP basis set with inclusion of solvent effects via conductor-like polarizable continuum model (CPCM) and as solvent acetonitrile. If more than one conformation is viable, the conformation with the lowest total energy is selected. The structures of all the molecules were optimized without symmetry or internal constrains and were verified as true minima by the absence of negative eigenvalues in the harmonic vibrational frequency analysis.

### HOMO and LUMO levels of the matrix compound and/or compound of formula (IV) or (V)

The HOMO and LUMO levels of the matrix compound and/or compound of formula (IV) or (V) are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

### Experimental data

Metal complexes and compounds of the present invention may be prepared using methods as described below or by methods known in the art.

### General method for the preparation of ligands HL

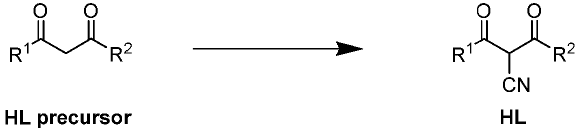

The **HL precursor** may be prepared by methods known in the art.

To a solution of **HL precursor** and K₂CO₃ in aqueous THF was added TsCN in one portion and the resulting mixture was stirred at RT for 3 h. The reaction mixture was concentrated under vacuum. The residue was stirred in DCM/water, the formed suspension was filtered, white solid was washed with 2x100 ml water and 100 ml DCM and dried. The product was stirred in 2M HCl / DCM mixture for 1 h. After separation of the layers, the organic phase was washed with 100 ml 2M HCl. Combined aqueous layers were washed with 200 ml DCM. Combined organic phases were washed with 300 ml water, dried over MgSO4, filtered and concentrated to give solid which was treated with hexane/diethyl ether. The solid was filtered off and dried in vacuum to yield **HL.**

| Chemical formula of **HL** | Yield [%] | Purity by HPLC [%] |
|---|---|---|
| 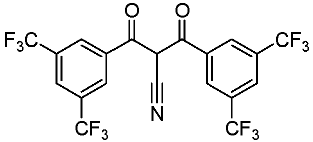 | 79 | > 98 |
| 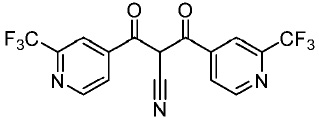 | 48 | > 98 |
| 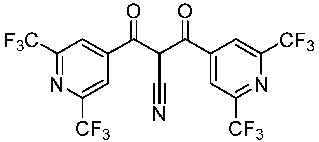 | 32 | > 95 |
| 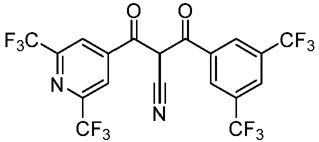 | 25 | > 95 |

### General method for preparation of metal complexes of formula (I)

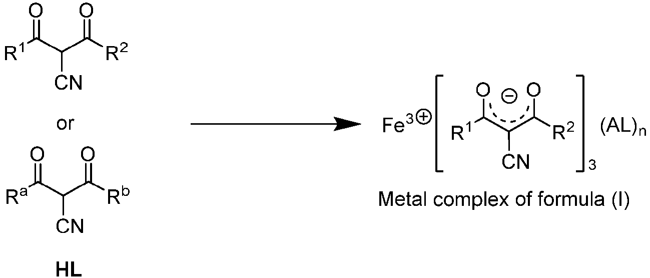

3 equivalents **HL** was dissolved in methanol and 3 equivalents sodium bicarbonate was added. 1 equivalent iron trichloride was dissolved in water and added to the mixture under cooling. The mixture was stirred at room temperature overnight. The precipitate was filtered off, washed with water and dried in high vacuum.

| Metal complex of formula (I) | Yield [%] | Purity by HPLC [%] |
|---|---|---|
| MC-1 | 24 | > 94 |
| MC-2 | 64 | > 98 |
| MC-3 | 80 | > 98 |
| MC-4 | 20 | > 94 |

The metal complexes of formula (I) may be purified further by methods known in the art prior to use in organic electronic devices, for example by distillation or sublimation in vacuum.

### General procedure for fabrication of OLEDs

### General method for preparation of the ink formulation

To prepare the ink formulation, the compounds were weighed into vials. Then, the solvent was added. The mixture was stirred for 10 min. The inks were transferred to inert atmosphere. An aliquot of benzonitrile solutions was added to the anisole solution to obtain a solution with a ratio of 5:1 of anisole to benzonitrile solution. The resulting solution was stirred again for at least 10 min at room temperature. The resulting ink formulation had a solid content of 4 wt.-%.

### Ink formulation for comparative example 1-1

The ink formulation for example 1-2 has the following composition: 4 wt.-% CC1 : 96 wt.- % K1 in anisole : benzonitrile (5:1). To prepare the ink, solutions of 12.4 mg (2 mol.-%, 2.35 wt. %) CC1 in 3 ml benzonitrile and 121 mg K1 in 3.3 ml anisole were prepared as described above. 0.7 ml benzonitrile solution was added to the anisole solution and stirred as described above.

### Ink formulation for inventive example 1-1

The ink formulation for example 1-x has the following composition: 4 wt.-% MC-2 : K1 in anisole : benzonitrile (5:1). To prepare the ink, solutions of 16.3 mg (2 mol. %, 4.63 wt.-%) MC-2 in 2 ml benzonitrile and 118 mg K1 in 3.3 ml anisole were prepared as described above. 0.7 ml benzonitrile solution was added to the anisole solution and stirred as described above.

### Ink formulation for inventive example 1-2

The ink formulation for example 1-1 has the following composition: 4 wt.-% MC-3 : 96 wt.-% K1 in anisole : benzonitrile (5:1). To prepare the ink, solutions of 12.4 mg (2 mol.-%, 3.52 wt. %) MC-3 in 2 ml benzonitrile and 119 mg K1 in 3.3 ml anisole were prepared as described above. 0.7 ml benzonitrile solution was added to the anisole solution and stirred as described above.

### General procedure for organic electronic devices wherein the semiconductor layer is deposited from solution

For inventive example 1-1 and 1-2 and comparative examples 1-1, see Table 2, a 15Ω /cm² glass substrate with 90 nm ITO (available from Corning Co.) with the dimensions 150 mm x 150 mm x 0.7 mm was ultrasonically washed with isopropyl alcohol for 5 minutes and then with pure water for 5 minutes and dried at elevated temperature.

Then, the metal complex and the matrix compound were co-deposited from solution on the anode layer, to form a hole injection layer (HIL) having a thickness of 50 nm. The formulae of the metal complexes can be seen in Table 1.

To form the hole injection layer, the substrate is placed on a spin-coater with ITO side facing upwards and fixed with vacuum. The ink is prepared as described above under general method. 4 ml of ink formulation is applied with a syringe with filter (PTFE - 0.2um) on the substrate. Spin-coating parameter are 850 rpm (3sec ramp-up from zero to maximum speed) for 30sec. The resulting film is dried at 60°C for 1 minute on a hotplate. Next step is the cleaning of the substrate around the active area (to ensure a good encapsulation before measurement). An additional bake-out at 100°C for 10 minutes on a hotplate is done. The composition of the hole injection layer can be seen in Table 2.

Then, the substrate is transferred to an evaporation tool for deposition of subsequent layers.

Then, N-([1,1'-biphenyl]-4-yl)-9,9-dimethyl-N-(4-(9-phenyl-9H-carbazol-3-yl)phenyl)-9H-fluoren-2-amine was vacuum deposited on the HIL, to form a HTL having a thickness of 88 nm.

Then N-(4-(dibenzo[b,d]furan-4-yl)phenyl)-N-(4-(9-phenyl-9H-fluoren-9-yl)phenyl)-[1,1'-biphenyl]-4-amine (CAS 1824678-59-2) was vacuum deposited on the HTL, to form an electron blocking layer (EBL) having a thickness of 5 nm.

Then 97 vol.-% H09 as EML host and 3 vol.-% BD200 (Sun Fine Chemicals, Korea) as fluorescent blue emitter dopant were deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then Al was evaporated at a rate of 0.01 to 1 Å/s at 10⁻⁷ mbar to form a cathode layer with a thickness of 100 nm on the electron transporting layer.

The OLED stack is protected from ambient conditions by encapsulation of the device with a glass slide. Thereby, a cavity is formed, which includes a getter material for further protection.

### General procedure for fabrication of organic electronic devices wherein the semiconductor layer is deposited in vacuum

For inventive examples 2-1 to 2-16 and comparative example 2-1 to 2-5, see Table 3, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 97.6 vol.-% nitrogen and 2.4 vol.-% oxygen.

Then, the metal complex and the matrix compound were co-deposited in vacuum on the anode layer, to form a hole injection layer (HIL) having a thickness of 10 nm. The matrix compound may be a compound of formula (IV) or (V) The composition of the hole injection layer can be seen in Table 3. The formulae of the metal complexes can be seen in Table 1.

Then, the matrix compound was vacuum deposited on the HIL, to form a HTL having a thickness of 128 nm. The matrix compound in the HTL is selected the same as the matrix compound in the HIL. The matrix compound in the HIL can be seen in Table 3.

Then N,N-di([1,1'-biphenyl]-4-yl)-3'-(9H-carbazol-9-yl)-[1,1'-biphenyl]-4-amine (CAS 1464822-27-2) was vacuum deposited on the HTL, to form an electron blocking layer (EBL) having a thickness of 5 nm.

Then 99 vol.-% blue emitter host BH1 (CAS 2457172-82-4) and 1 vol.-% BD1 (CAS 2482607-57-6) as blue emitter dopant were co-deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer (ETL) having a thickness of 31 nm was formed on the hole blocking layer by depositing 50 wt.-% 2-(2',6'-diphenyl-[1,1':4',1"-terphenyl]-4-yl)-4-phenyl-6-(3-(pyridin-4-yl)phenyl)-1,3,5-triazine and 50 wt.-% of LiQ.

Then Yb was deposited on the ETL to form an electron injection layer (EIL) having a thickness of 2 nm.

Then Ag:Mg (90:10 vol.-%) was evaporated at a rate of 0.01 to 1 Å/s at 10⁻⁷ mbar to form a cathode layer with a thickness of 13 nm on the electron injection layer.

Then, N-([1,1'-biphenyl]-4-yl)-9,9-dimethyl-N-(4-(9-phenyl-9H-carbazol-3-yl)phenyl)-9H-fluoren-2-amine was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

In bottom emission devices, the emission is predominately Lambertian and quantified in percent external quantum efficiency (EQE). The light is emitted through the anode layer. To determine the efficiency EQE in % the light output of the device is measured using a calibrated photodiode at 10 mA/cm2.

In top emission devices, the emission is forward directed through the cathode layer, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm².

Lifetime LT of the device is measured at ambient conditions (20°C) and 30 mA/cm², using a Keithley 2400 source meter, and recorded in hours.

The brightness of the device is measured using a calibrated photo diode. The lifetime LT is defined as the time till the brightness of the device is reduced to 97 % of its initial value.

To determine the voltage stability over time U(100h)-(1h), a current density of at 30 mA/cm² was applied to the device. The operating voltage was measured after 1 hour and after 100 hours, followed by calculation of the voltage stability for the time period of 1 hour to 100 hours. A low value for U(100h)-(1h) denotes a low increase in operating voltage over time and thereby improved voltage stability.

### Technical Effect of the invention

In Table 1 are shown the calculated LUMO in electron volt of metal complexes of formula (I). Where more than one spin state is viable, the spin state is stated in brackets. As can be seen in Table 1, the LUMO levels of metal complexes of formula (I) are within the range suitable for organic electronic devices.

Also in Table 1 are shown calculated LUMO in electron volt of comparative compounds CC1 and CC2. The same method was used as for metal complexes of formula (I).

Comparative compound CC1 has the formula below:

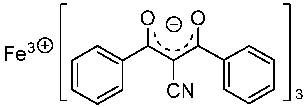

Comparative compound CC1 is lacking at least two CF₃ groups and/or at least one N atom. As can be seen in Table 1, the LUMO level is closer to vacuum level than LUMO energies of metal complexes of formula (I).

Comparative compound CC2 has the formula below:

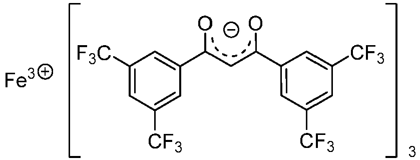

Comparative compound CC2 is lacking the CN group in formula (II). As can be seen in Table 1, the LUMO level is closer to vacuum level than LUMO energies of metal complexes of formula (I).

Without being bound by theory, a LUMO energies further away from vacuum level is beneficial for improved performance of organic electronic devices.

**Table 1: LUMO values of metal complexes according to Formula (I) and comparative compounds 1 and 2**

| | Name | Structure (if not already disclosed) | LUMO [eV] (spin) |
|---|---|---|---|
| Comparative compound 1 | CC1 | | -4.09eV (β) |
| Comparative compound 2 | CC2 | | -3.92eV (β) |
| Inventive compound 1 | MC-1 | L52 | -4.67eV (β) |
| Inventive compound 2 | MC-2 | L1 | -4.48eV (β) |
| Inventive compound 3 | MC-3 | L9 | -4.63eV (β) |
| Inventive compound 4 | MC-4 | L10 | -4.80eV (β) |
| Inventive compound 5 | MC-5 | L11 | -4.63eV (β) |

In Table 2 are shown data for bottom emission organic electronic devices fabricated by co-deposition from solution of a composition comprising metal complex of formula (I) or comparative compound CC1 and matrix compound K1.

In comparative example 1-1, a metal complex known in the art is tested at 2 mol.- %. As can be seen in Table 2, in comparative example 1-1 the operating voltage is 6.63 V, the external quantum efficiency EQE is less than 5 %. Due to the very high operating voltage and very poor efficiency, the lifetime and voltage stability over time were not determined.

In inventive example 1-1, solution processing of a composition comprising metal complex of formula (I) MC-2 and matrix compound K1 was attempted. However, no layer was formed. Therefore, this metal complex was deposited from vacuum in subsequent inventive examples, see Table 3.

In inventive example 1-2, a composition comprising metal complex of formula (I) MC-3 and matrix compound K1 was assessed. As can be seen in Table 2, the operating voltage is improved to 3.65 V, cd/A the EQE is improved to 10.28 %, the lifetime is improved to 119 hours and operating voltage stability over time is improved to 0.404 V.

In Table 3 are shown data for top emission organic electronic devices fabricated by co-deposition from vacuum of the metal complex and the matrix compound.

In comparative example 2-1 to 2-5, a second metal complex known in the art is tested at concentrations in the range of 8 to 18 wt.-%.

As can be seen in Table 3, in comparative examples 2-1 to 2-5 the operating voltage is in the range of 3.52 to 3.54 V, the cd/A efficiency is in the range of 9.76 to 10.15 cd/A, the external quantum efficiency EQE is in the range of 19.59 to 20.42 % and the voltage stability over time is in the range of 1.52 to 2.67 V.

In inventive example 2-1, the semiconductor layer comprises a metal complex of formula (I) MC-2. The matrix compound is the same as in comparative examples 2-1 to 2-5. The concentration of metal complex of formula (I) in the layer is 1.5 wt.-% As can be seen in Table 3, the operating voltage is improved to 3.47 V, cd/A efficiency is 10.52 cd/A, the EQE is 20.73 %, the lifetime is 82 hours and operating voltage stability over time is improved to 0.11 V.

In inventive examples 2-2 to 2-7, the concentration of metal complex of formula (I) MC-2 has been varied from 2 to 14 wt.-%. As can be seen in Table 3, the operating voltage, is improved to 3.4 to 3.44 V compared to comparative examples 2-1 to 2-5. The cd/A efficiency is improved to 10.27 to 10.65 cd/A. The EQE is improved to 20.31 to 20.73 %, the lifetime is in the range of 71 to 91 hours. The voltage stability over time is improved substantially to 0.04 to 0.11 V.

In inventive examples 2-8 to 2-11, the semiconductor layer comprises matrix compound K7. K7 has a HOMO level further away from vacuum level, namely -4.84 eV compared to -4.73 for K16. The concentration of metal complex of formula (I) MC-2 has been varied from 10 to 16 wt.-%. As can be seen in Table 3, the operating voltage, is improved to 3.38 to 3.44 V compared to comparative examples 2-1 to 2-5. The cd/A efficiency and EQE are lower than in comparative examples 2-1 to 2.5. However, the lifetime is substantially improved to 159 to 215 hours. The voltage stability over time is improved substantially to 0.07 to 0.18 V.

In inventive examples 2-12 to 2-16, the semiconductor layer comprises matrix compound K2. K2 has a HOMO level further away from vacuum level, namely -4.85 eV compared to -4.73 for K16. The concentration of metal complex of formula (I) MC-2 has been varied from 4 to 19 wt.-%. As can be seen in Table 3, the operating voltage, is improved to 3.33 to 3.35 V compared to comparative examples 2-1 to 2-5. The cd/A efficiency and EQE are in a similar range to comparative examples 2-1 to 2.5. However, the lifetime is improved to 92 to 114 hours. The voltage stability over time is improved substantially to 0.03 to 0.21 V.

A low operating voltage, high efficiency, high lifetime and/or improved operating voltage stability over time are important for the performance and long-term stability of organic electronic devices.

**Table 2: Performance of an organic electroluminescent device prepared via deposition of the semiconductor layer from solution**

| | Metal comple x | Percentage metal complex in semiconducto r layer [wt.-%] | Matrix compoun d | HOMO level of matrix compoun d [eV] | Percentage matrix compound in semiconducto r layer [wt.-%] | Semiconducto r layer thickness [nm] | Voltage at 10mA/cm ² [V] | EQE at 10mA/cm ²[%] | LT97 at 30mA/cm ² [h] | U(100h)-(1h) at 30mA/cm ² [V] |
|---|---|---|---|---|---|---|---|---|---|---|
| Comparativ e example 1-1 | CC1 | 4 | K1 | -4.68 | 96 | 50 | 6.63 | < 5 | n.d. ¹ | n.d |
| Inventive example 1-1 | MC-2 | 4 | K1 | -4.68 | 96 | No layer formed | n.d. | n.d. | n.d. | n.d. |
| Inventive example 1-2 | MC-3 | 4 | K1 | -4.68 | 96 | 50 | 3.65 | 10.28 | 119 | 0.404 |

| | | | | | | | | | | |
|---|---|---|---|---|---|---|---|---|---|---|
| ¹ "n.d." = not determined | | | | | | | | | | |

**Table 3: Performance of an organic electroluminescent device prepared via deposition of the semiconductor layer in vacuum**

| | Metal complex | Percentage metal complex in semiconduct or layer [wt.-%] | Matrix compoun d | HOMO level of matrix compoun d [eV] | Percentage matrix compound in semiconducto r layer [wt.-%] | Voltage at 10mA/cm ²[V] | Cd/A efficiency at 10mA/cm ²[cd/A] | EQE at 10mA/cm ²[%] | LT97 at 30mA/cm ² [h] | U(100h)-(1h) at 30mA/cm ² [V] |
|---|---|---|---|---|---|---|---|---|---|---|
| Comparative example 2-1 | CC2 | 8 | K16 | -4.73 | 92 | 3.54 | 9.84 | 19.87 | 72 | 1.67 |
| Comparative example 2-2 | CC2 | 10 | K16 | -4.73 | 90 | 3.53 | 9.98 | 19.77 | 71 | 1.52 |
| Comparative example 2-3 | CC2 | 13 | K16 | -4.73 | 87 | 3.52 | 9.76 | 19.59 | 75 | 1.73 |
| Comparative example 2-4 | CC2 | 16 | K16 | -4.73 | 84 | 3.54 | 10.15 | 20.42 | 85 | 2.67 |
| Comparative example 2-5 | CC2 | 18 | K16 | -4.73 | 82 | 3.53 | 10.09 | 20.38 | 81 | 2.55 |
| Comparative example 2-6 | CC2 | 23 | K16 | -4.73 | 77 | 3.52 | 9.99 | 20.38 | 81 | 2.34 |
| Inventive example 2-1 | MC-2 | 1.5 | K16 | -4.73 | 98.5 | 3.47 | 10.52 | 20.73 | 82 | 0.11 |
| Inventive example 2-2 | MC-2 | 2 | K16 | -4.73 | 98 | 3.44 | 10.44 | 20.66 | 80 | 0.08 |
| Inventive example 2-3 | MC-2 | 4 | K16 | -4.73 | 96 | 3.42 | 10.65 | 20.53 | 70 | 0.05 |
| Inventive example 2-4 | MC-2 | 6 | K16 | -4.73 | 94 | 3.40 | 10.52 | 20.54 | 71 | 0.04 |
| Inventive example 2-5 | MC-2 | 9 | K16 | -4.73 | 91 | 3.40 | 10.41 | 20.39 | 91 | 0.06 |
| Inventive example 2-6 | MC-2 | 11 | K16 | -4.73 | 89 | 3.40 | 10.27 | 20.41 | 82 | 0.05 |
| Inventive example 2-7 | MC-2 | 14 | K16 | -4.73 | 86 | 3.40 | 10.27 | 20.31 | 83 | 0.05 |
| Inventive example 2-8 | MC-2 | 10 | K7 | -4.84 | 90 | 3.38 | 8.45 | 18.00 | 172 | 0.18 |
| Inventive example 2-9 | MC-2 | 12 | K7 | -4.84 | 88 | 3.44 | 8.88 | 18.16 | 214 | 0.13 |
| Inventive example 2-10 | MC-2 | 14 | K7 | -4.84 | 86 | 3.43 | 8.87 | 18.18 | 178 | 0.09 |
| Inventive example 2-11 | MC-2 | 16 | K7 | -4.84 | 84 | 3.42 | 8.87 | 18.20 | 159 | 0.07 |
| Inventive example 2-12 | MC-2 | 8 | K2 | -4.85 | 92 | 3.35 | 9.90 | 19.81 | 113 | 0.21 |
| Inventive example 2-13 | MC-2 | 10 | K2 | -4.85 | 90 | 3.35 | 9.76 | 19.45 | 114 | 0.17 |
| Inventive example 2-14 | MC-2 | 12 | K2 | -4.85 | 88 | 3.33 | 9.88 | 19.81 | 107 | 0.11 |
| Inventive example 2-15 | MC-2 | 15 | K2 | -4.85 | 85 | 3.34 | 9.76 | 19.50 | 93 | 0.05 |
| Inventive example 2-16 | MC-2 | 19 | K2 | -4.85 | 81 | 3.34 | 9.64 | 19.46 | 92 | 0.03 |

The particular combinations of elements and features in the above detailed embodiments are exemplary only. As those skilled in the art will recognize, variations, modifications, and other implementations of what is described herein can occur to those of ordinary skill in the art without departing from the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

## Claims

1. A metal complex of formula (I) 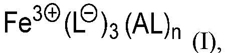 wherein
L has formula (II) 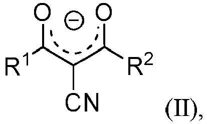
whereby
R¹ is selected from substituted C₂ to C₁₂ alkyl, substituted C₆ to C₁₉ aryl, substituted or unsubstituted C₂ to C₂₀ heteroaryl, or substituted or unsubstituted 6-membered heteroaryl; R² is selected from substituted C₆ to C₁₉ aryl, substituted or unsubstituted C₂ to C₂₀ heteroaryl, substituted or unsubstituted 6-membered heteroaryl;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
L comprises at least two CF₃ groups and/or at least one N atom;
and wherein
AL is an ancillary ligand which coordinates to the metal M;
n is an integer selected from 0 to 2.

2. The metal complex of formula (I) according to claim 1 , whereby R¹ is selected from substituted C₆ to C₁₉ aryl, substituted or unsubstituted C₂ to C₂₀ heteroaryl, or substituted or unsubstituted 6-membered heteroaryl.

3. The metal complex of formula (I) according to Claim 1 or 2, wherein R¹ and R² are identical.

4. The metal complex of formula (I) according to any of the claims 1 to 3, wherein R¹ and/or R² are selected from one of the following groups D1 to D70 (in consideration of the above provisos for L): 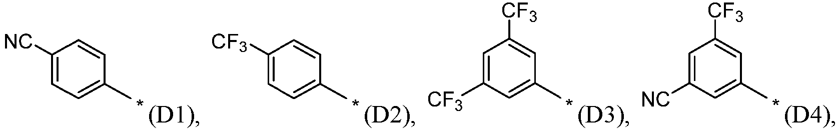 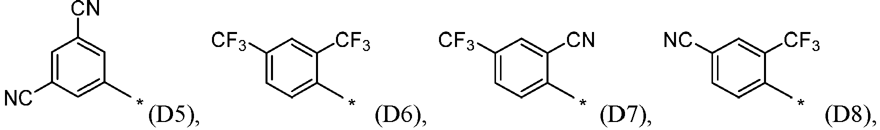 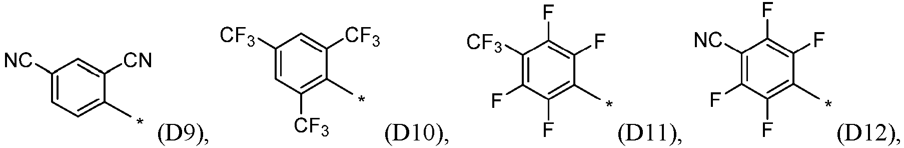 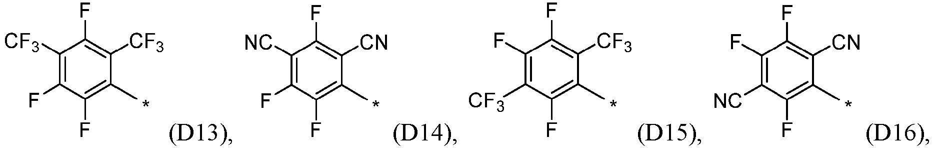 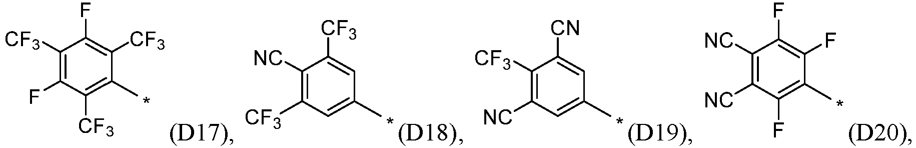 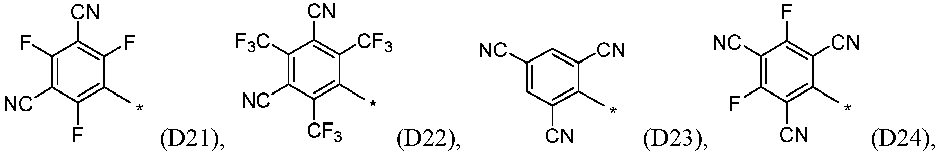 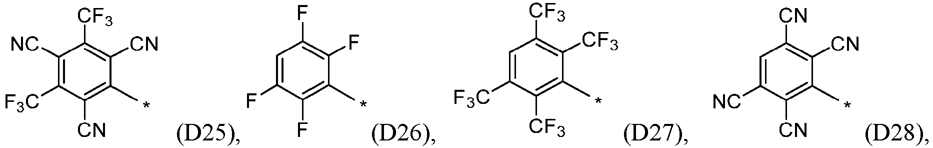 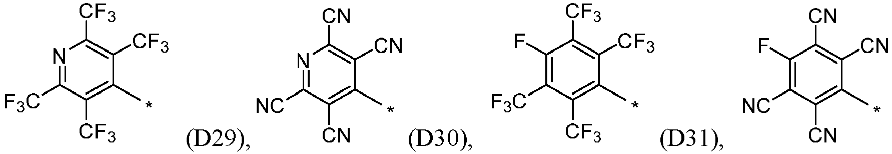 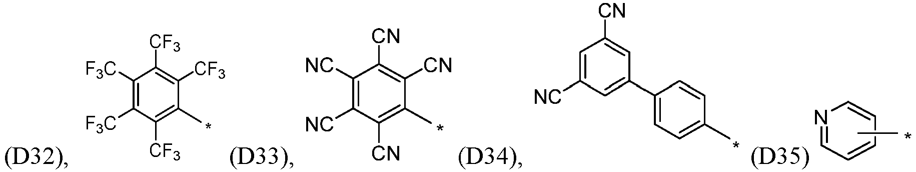 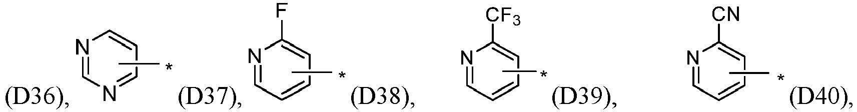 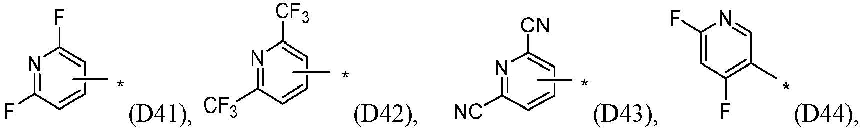 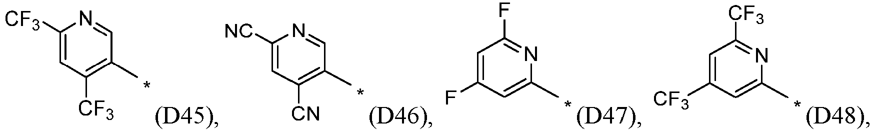 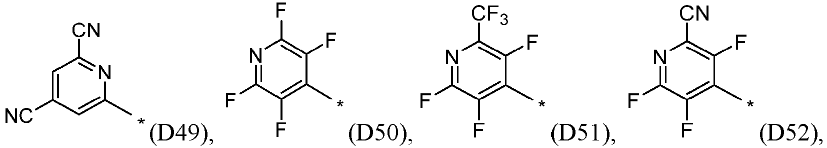 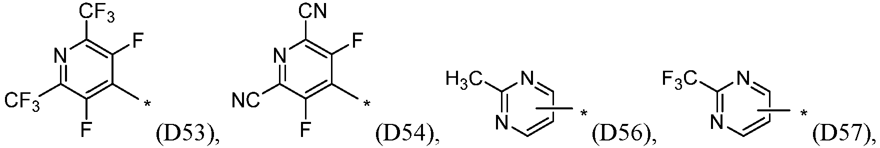 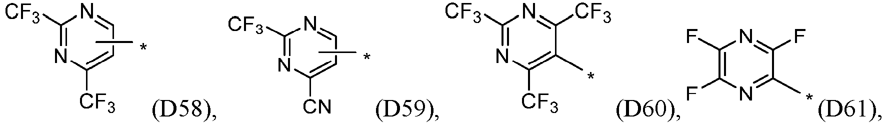 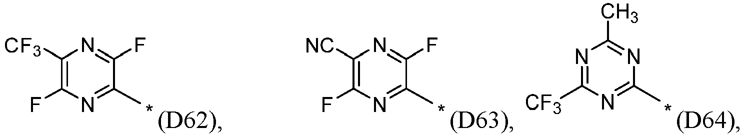 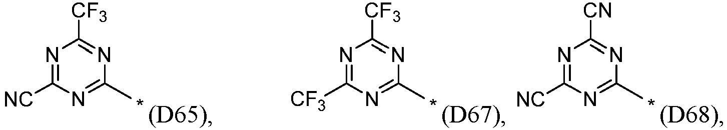 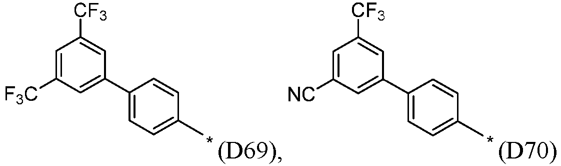 wherein the "*" denotes the binding position.

5. The metal complex of formula (I) according to any of the claims 1 to 4, wherein the ligand L is selected from one of the following groups L1 to L54:

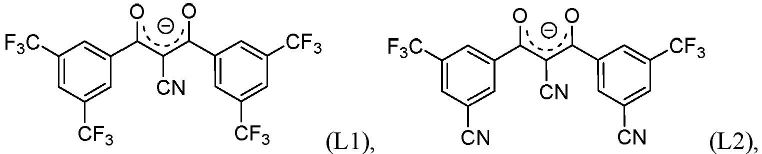

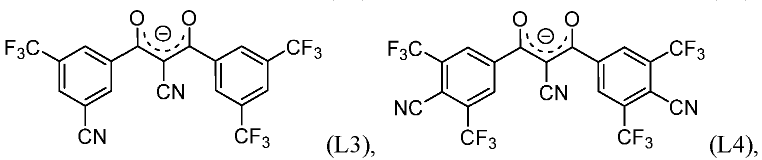

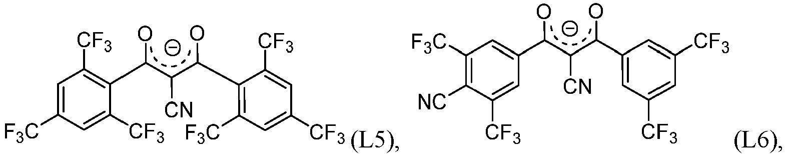

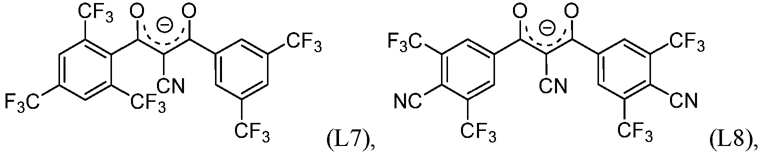

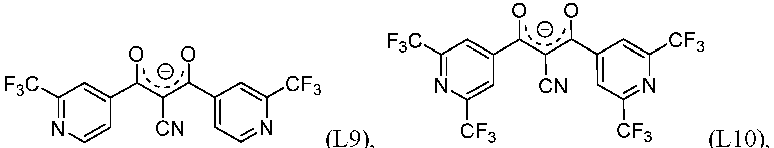

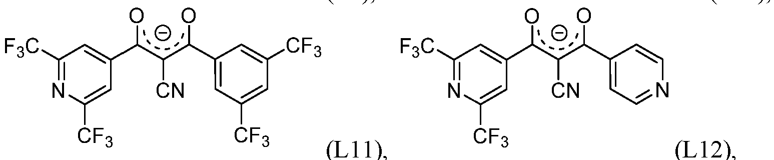

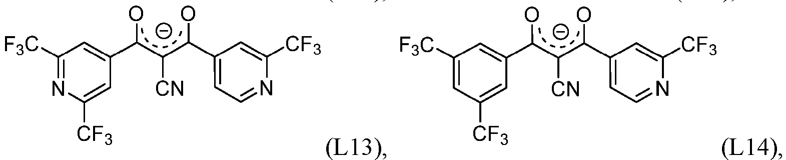

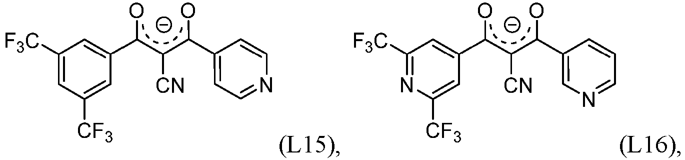

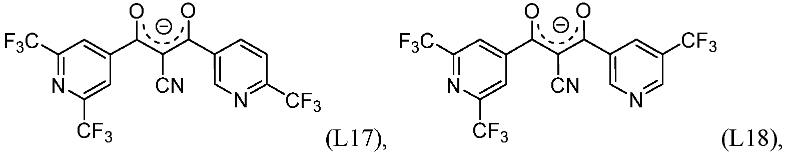

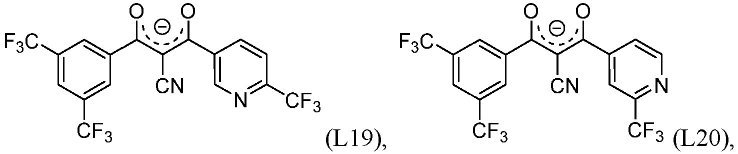

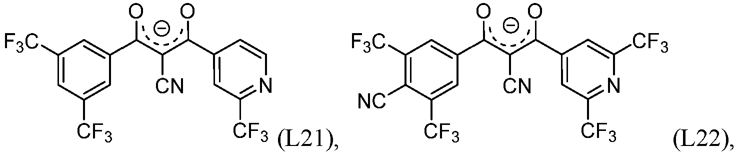

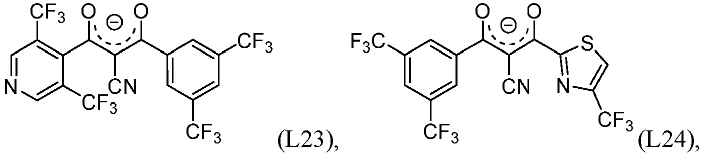

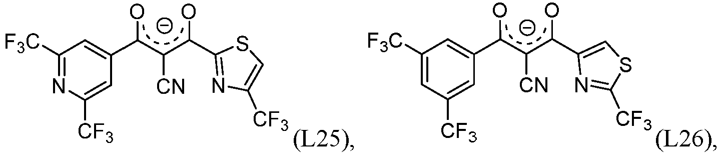

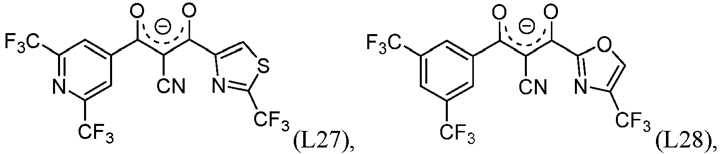

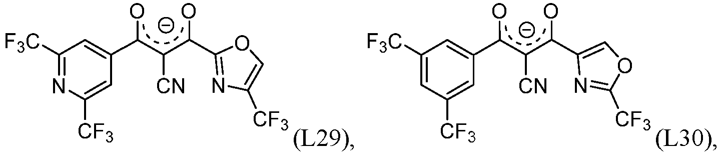

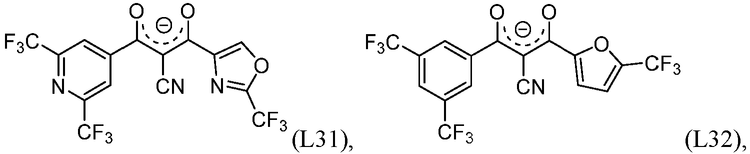

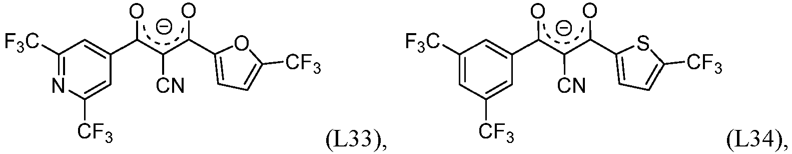

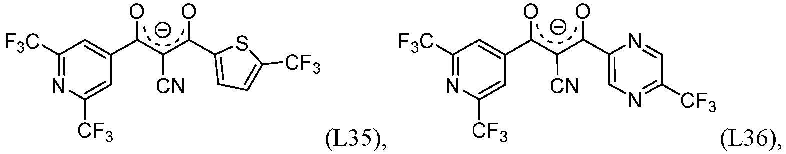

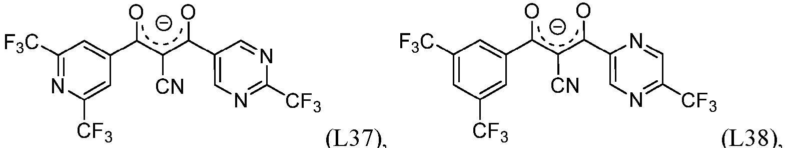

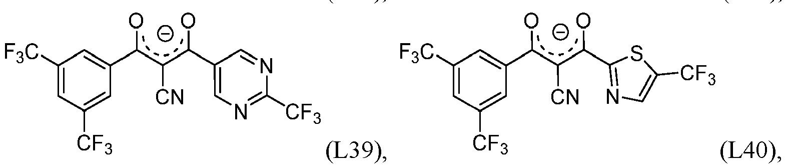

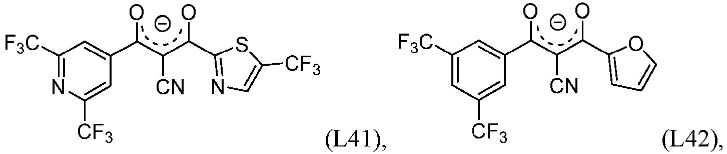

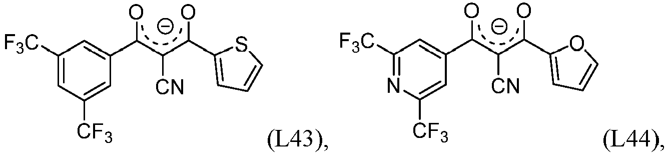

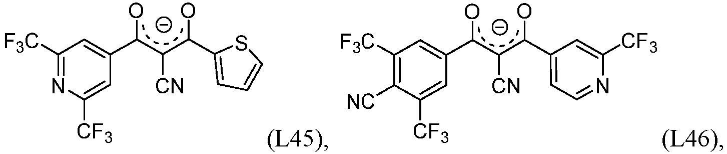

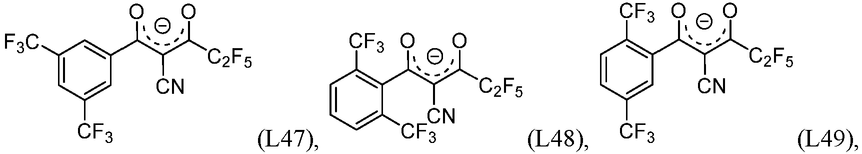

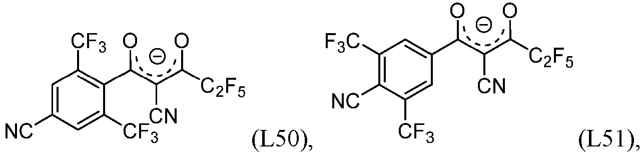

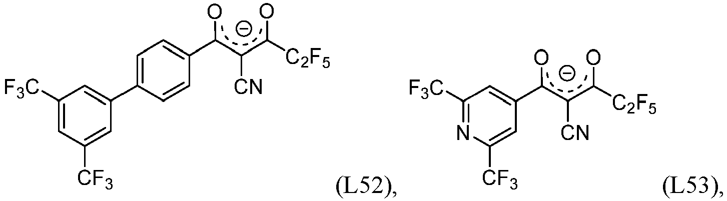

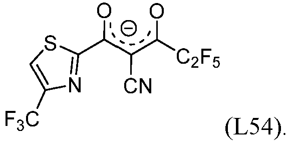

6. An organic semiconductor layer, whereby the organic semiconductor layer comprises a metal complex of formula (I) of any of the preceding claims 1 to 5.

7. An organic electronic device comprising an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to claim 6.

8. An organic electronic device according to claim 7, whereby the anode layer comprises at least a first anode sub-layer and a second anode sub-layer.

9. The organic electronic device of claim 7 or 8, whereby the organic electronic device further comprises at least one photoactive layer, wherein the at least one photoactive layer is arranged between the anode layer and the cathode layer; preferably at least one of the at least one organic semiconductor layers is arranged between the anode layer and the at least one photoactive layer.

10. The organic electronic device according to any of the preceding claims 7 to 9, whereby the photoactive layer is a light emitting layer.

11. The organic electronic device of any of the preceding claims 7 to 10, whereby the organic electronic device is an electroluminescent device, an organic light emitting diode (OLED), a light emitting device, thin film transistor, a battery, a display device or an organic photovoltaic cell (OPV).

12. A display device comprising an organic electronic device according to any of the preceding claims 7 to 11.

13. A compound of formula (IIa) 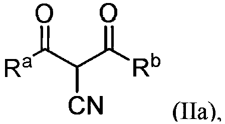 whereby
R^{a} is selected from substituted C₂ to C₁₂ alkyl, substituted C₆ to C₁₉ aryl, substituted or unsubstituted C₂ to C₂₀ heteroaryl, or substituted or unsubstituted 6-membered heteroaryl;
R^{b} is selected from the formulae D38 to D68 as defined in claim 4;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
the compound comprises at least two CF₃ groups and/or at least one N atom.

14. The compound of Claim 13, whereby R^{a} is selected from substituted C₂ to C₁₂ alkyl and substituted C₆ to C₁₉ aryl and R^{b} is selected from formulae D39, D42, D45, D48, D56, D57, D58, D67.

## Patentansprüche

1. Metallkomplex der Formel (I) 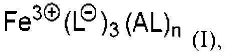 wobei
L die Formel (II) aufweist: 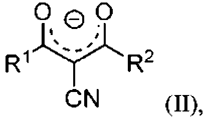
wobei
R¹ aus substituiertem C₂- bis C₁₂-Alkyl, substituiertem C₆- bis C₁₉-Aryl, substituiertem oder unsubstituiertem C₂-bis C₂₀-Heteroaryl oder substituiertem oder unsubstituiertem 6-gliedrigem Heteroaryl ausgewählt ist;
R² aus substituiertem C₆- bis C₁₉-Aryl, substituiertem oder unsubstituiertem C₂- bis C₂₀-Heteroaryl, substituiertem oder unsubstituiertem 6-gliedrigem Heteroaryl ausgewählt ist;
wobei
mindestens einer der Substituenten des substituierten C₁-bis C₁₂-Alkyls, substituierten C₆- bis C₁₉-Aryls, substituierten C₂- bis C₂₀-Heteroaryls oder substituierten 6-gliedrigen Heteroaryls unabhängig aus Halogen, Cl, F, CN, teil- oder perfluoriertem C₁- bis C₈-Alkyl, teil- oder perfluoriertem C₁- bis C₈-Alkoxy ausgewählt sind;
wobei
L mindestens zwei CF₃-Gruppen und/oder mindestens ein N-Atom umfasst;
und wobei
AL für einen Hilfsliganden steht, der an das Metall M koordiniert;
n für eine ganze Zahl von 0 bis 2 steht.

2. Metallkomplex der Formel (I) nach Anspruch 1, wobei R¹ aus substituiertem C₆- bis C₁₉-Aryl, substituiertem oder unsubstituiertem C₂- bis C₂₀-Heteroaryl oder substituiertem oder unsubstituiertem 6-gliedrigem Heteroaryl ausgewählt ist.

3. Metallkomplex der Formel (I) nach Anspruch 1 oder 2, wobei R¹ und R² gleich sind.

4. Metallkomplex der Formel (I) nach einem der Ansprüche 1 bis 3, wobei R¹ und/oder R² aus den folgenden Gruppen D1-D70 ausgewählt sind (unter Berücksichtigung der obigen Maßgaben für L): 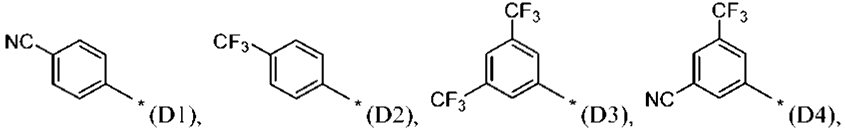 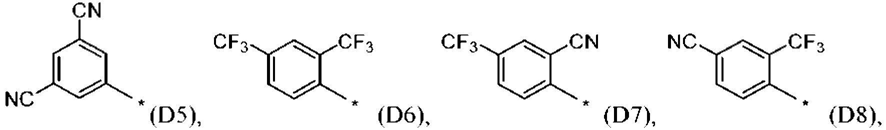 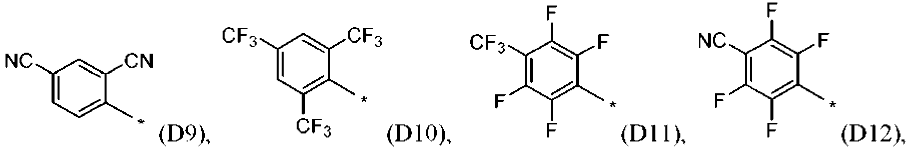 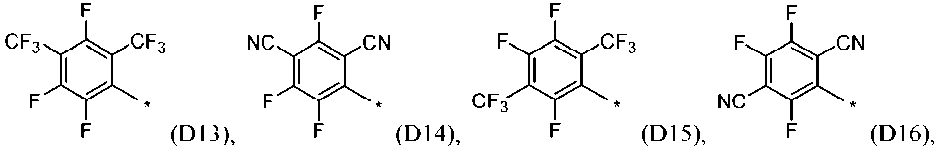 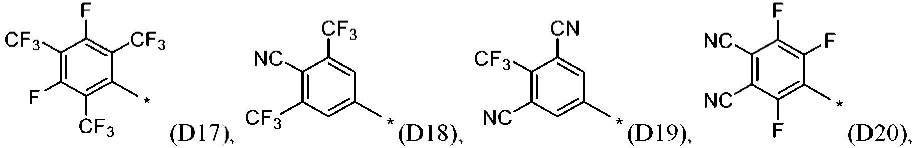 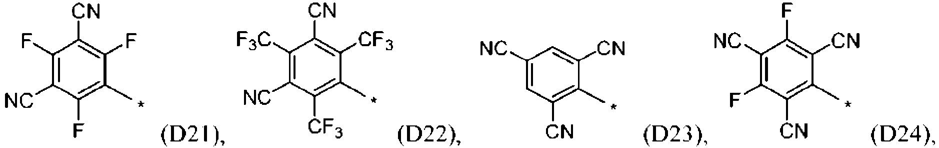 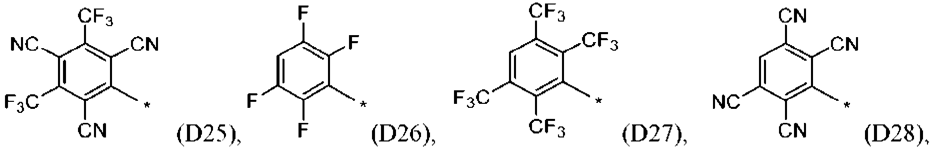 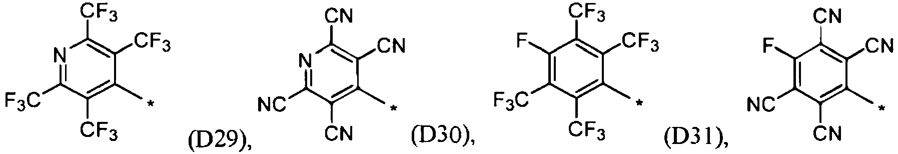 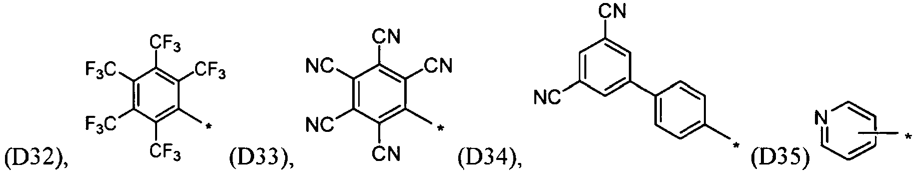 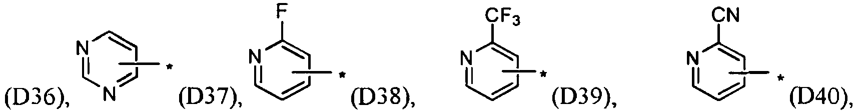 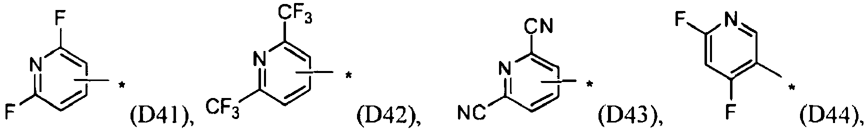 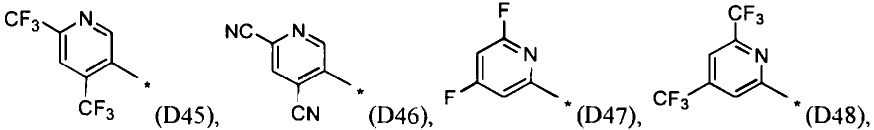 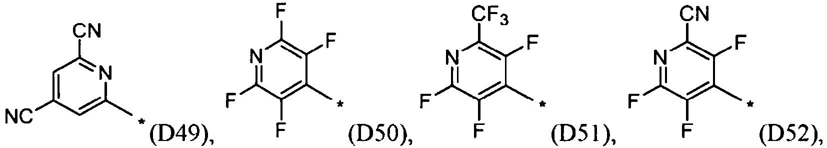 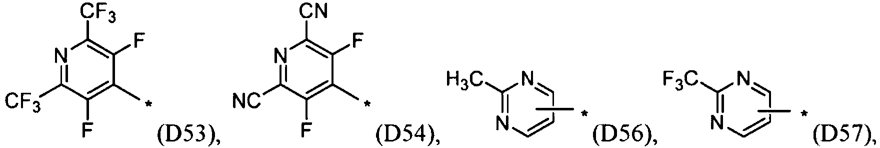 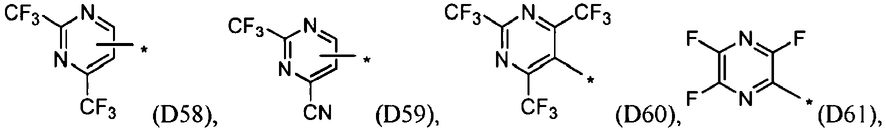 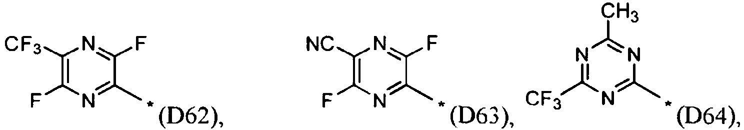 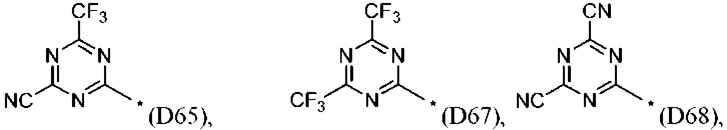 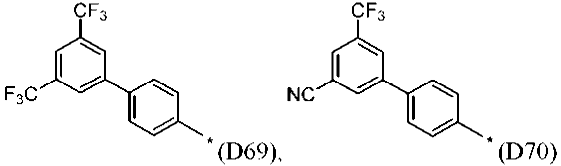 wobei das "*" die Bindungsposition bezeichnet.

5. Metallkomplex der Formel (I) nach einem der Ansprüche 1 bis 4, wobei der Ligand L aus einer der folgenden Gruppen L1 bis L54 ausgewählt ist:

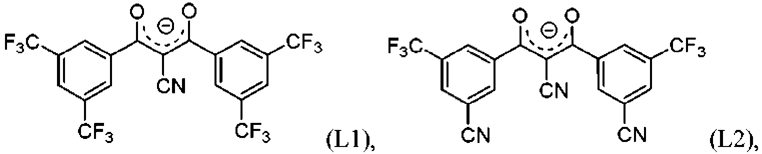

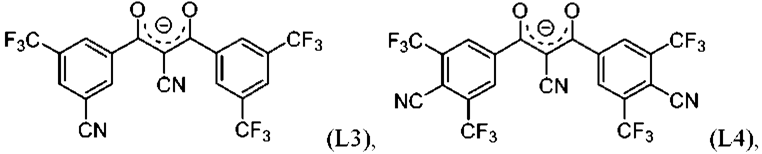

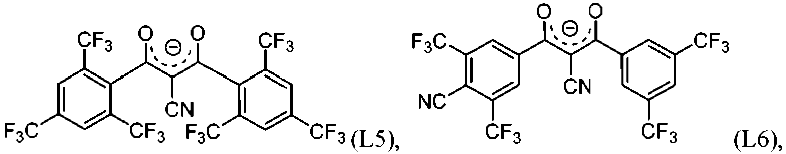

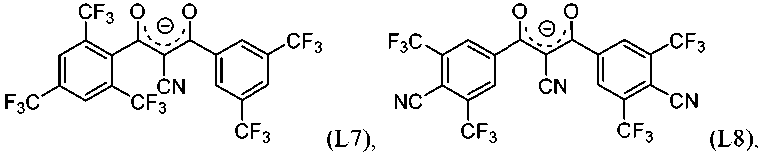

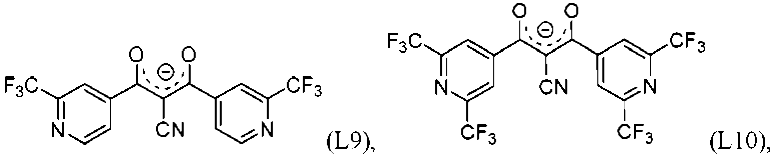

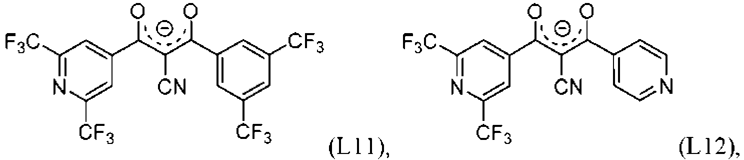

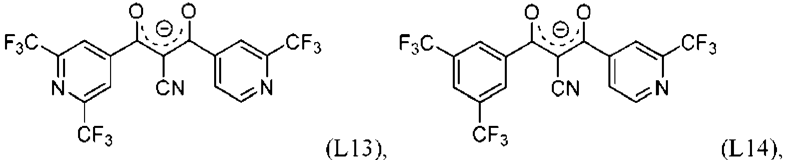

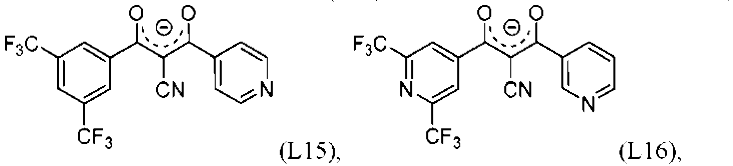

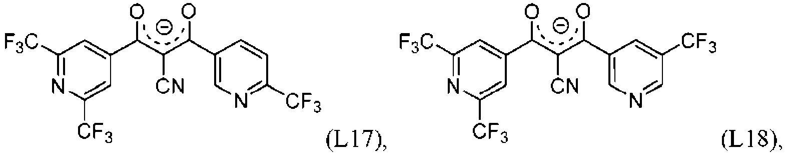

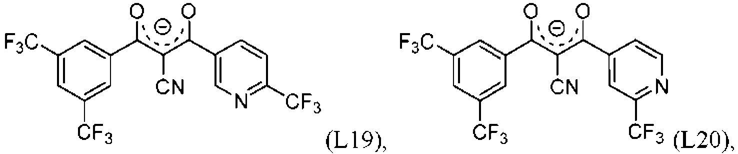

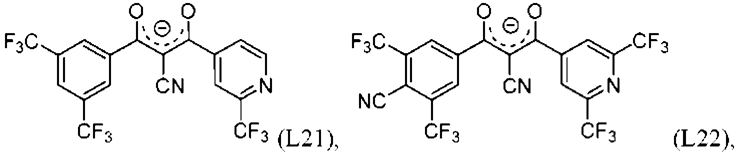

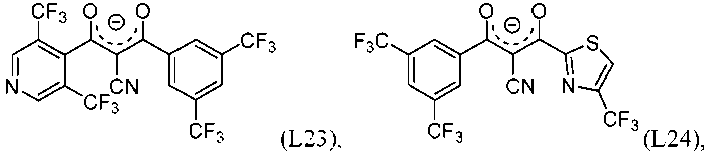

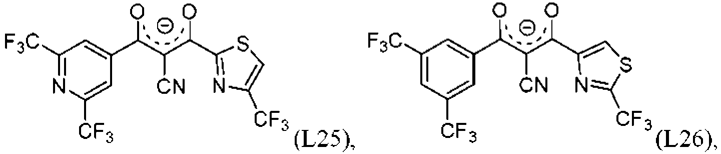

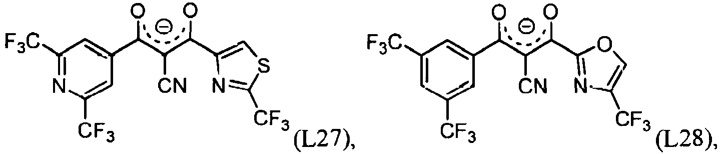

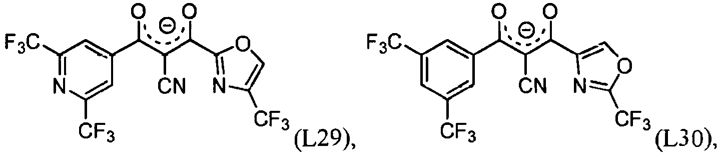

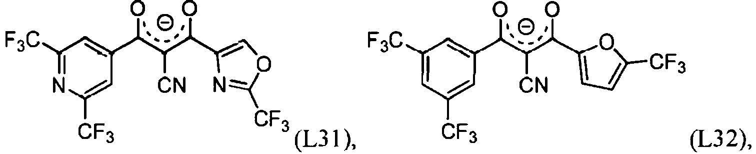

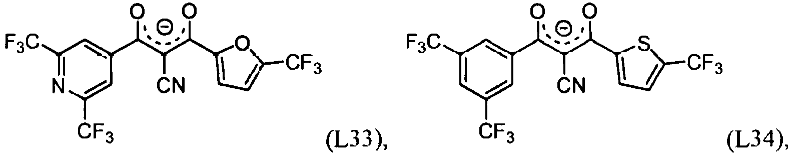

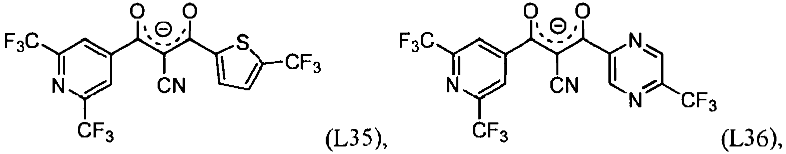

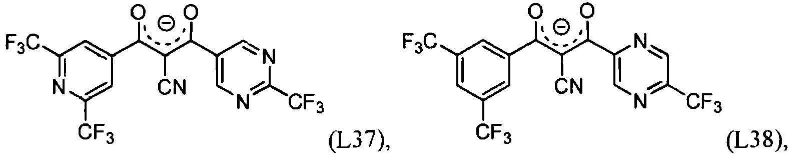

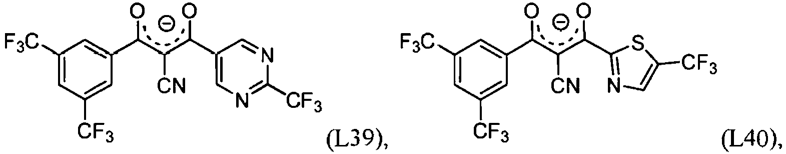

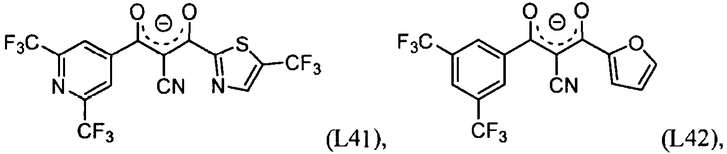

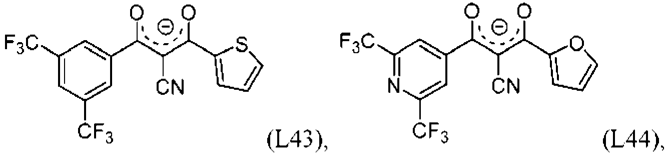

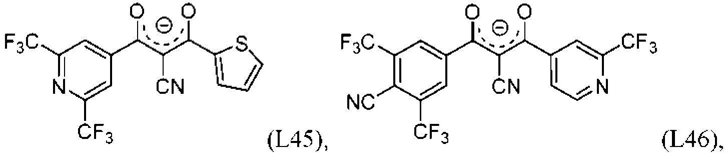

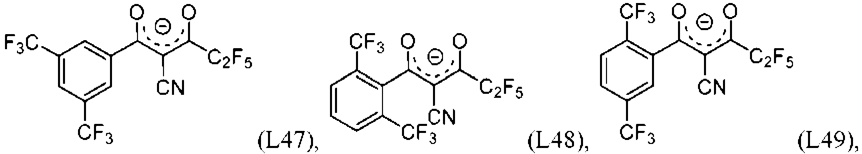

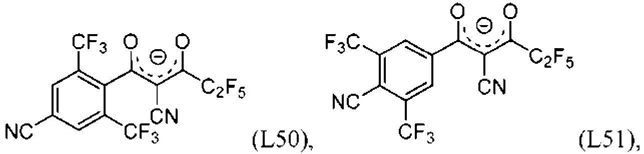

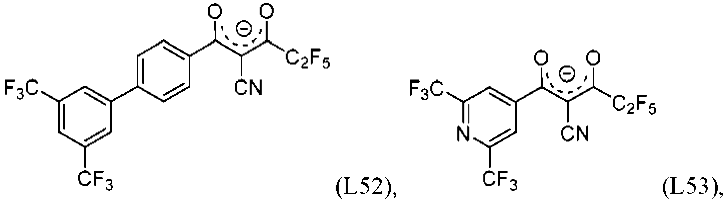

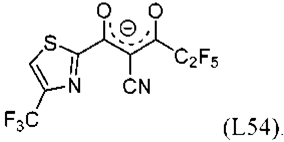

6. Organische Halbleiterschicht, wobei die organische Halbleiterschicht einen Metallkomplex der Formel (I) nach einem der vorhergehenden Ansprüche 1 bis 5 umfasst.

7. Organische elektronische Vorrichtung, umfassend eine Anodenschicht, eine Kathodenschicht und mindestens eine organische Halbleiterschicht, wobei die mindestens eine organische Halbleiterschicht zwischen der Anodenschicht und der Kathodenschicht angeordnet ist und wobei es sich bei der mindestens einen organischen Halbleiterschicht um die organische Halbleiterschicht gemäß Anspruch 6 handelt.

8. Organische elektronische Vorrichtung nach Anspruch 7, wobei die Anodenschicht mindestens eine erste Anoden-Unterschicht und eine zweite Anoden-Unterschicht umfasst.

9. Organische elektronische Vorrichtung nach Anspruch 7 oder 8, wobei die organische elektronische Vorrichtung ferner mindestens eine photoaktive Schicht umfasst, wobei die mindestens eine photoaktive Schicht zwischen der Anodenschicht und der Kathodenschicht angeordnet ist; vorzugsweise mindestens eine der mindestens einen organischen Halbleiterschichten zwischen der Anodenschicht und der mindestens einen photoaktiven Schicht angeordnet ist.

10. Organische elektronische Vorrichtung nach einem der vorhergehenden Ansprüche 7 bis 9, wobei es sich bei der photoaktiven Schicht um eine lichtemittierende Schicht handelt.

11. Organische elektronische Vorrichtung nach einem der vorhergehenden Ansprüche 7 bis 10, wobei es sich bei der organischen elektronischen Vorrichtung um eine Elektrolumineszenzvorrichtung, eine organische Leuchtdiode (OLED), eine lichtemittierende Vorrichtung, einen Dünnschichttransistor, eine Batterie, eine Anzeigevorrichtung oder eine organische photovoltaische Zelle (OPV) handelt.

12. Anzeigevorrichtung, umfassend eine organische elektronische Vorrichtung nach einem der vorhergehenden Ansprüche 7 bis 11.

13. Verbindung der Formel (IIa) 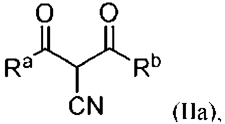 wobei
R^{a} aus substituiertem C₂- bis C₁₂-Alkyl, substituiertem C₆- bis C₁₉-Aryl, substituiertem oder unsubstituiertem C₂-bis C₂₀-Heteroaryl oder substituiertem oder unsubstituiertem 6-gliedrigem Heteroaryl ausgewählt ist; R^{b} aus den Formeln D38 bis D68 gemäß Anspruch 4 ausgewählt ist;
wobei
mindestens einer der Substituenten des substituierten C₁-bis C₁₂-Alkyls, substituierten C₆- bis C₁₉-Aryls, substituierten C₂- bis C₂₀-Heteroaryls oder substituierten 6-gliedrigen Heteroaryls unabhängig aus Halogen, Cl, F, CN, teil- oder perfluoriertem C₁- bis C₈-Alkyl, teil- oder perfluoriertem C₁- bis C₈-Alkoxy ausgewählt sind;
wobei
die Verbindung L mindestens zwei CF₃-Gruppen und/oder mindestens ein N-Atom umfasst.

14. Verbindung nach Anspruch 13, wobei R^{a} aus substituiertem C₂- bis C₁₂-Alkyl und substituiertem C₆-bis C₁₉-Aryl ausgewählt ist und R^{b} aus den Formeln D39, D42, D45, D48, D56, D57, D58, D67 ausgewählt ist.

## Revendications

1. Complexe métallique de formule (I) 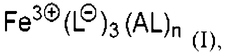 L ayant la formule (II) 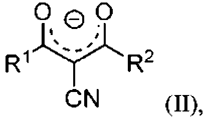
R¹ étant choisi parmi alkyle en C₂ à C₁₂ substitué, aryle en C₆ à C₁₉ substitué, hétéroaryle en C₂ à C₂₀ substitué ou non substitué, hétéroaryle à 6 chaînons substitué ou non substitué ;
R² étant choisi parmi aryle en C₆ à C₁₉ substitué, hétéroaryle en C₂ à C₂₀ substitué ou non substitué, hétéroaryle à 6 chaînons substitué ou non substitué ;
au moins un des substituants de l'alkyle en C₁ à C₁₂ substitué, l'aryle en C₆ à C₁₉ substitué, l'hétéroaryle en C₂ à C₂₀ substitué, ou l'hétéroaryle à 6 chaînons substitué étant indépendamment choisis parmi halogène, Cl, F, CN, alkyle en C₁ à C₈ partiellement fluoré ou perfluoré, alcoxy en C₁ à C₈ partiellement fluoré ou perfluoré ;
L comprenant au moins deux groupes CF₃ et/ou au moins un atome de N ;
et,
AL étant un ligand auxiliaire qui forme une liaison de coordination avec le métal M ;
n étant un entier choisi parmi 0 à 2.

2. Complexe métallique de formule (I) selon la revendication 1, dans lequel R¹ est choisi parmi aryle en C₆ à C₁₉ substitué, hétéroaryle en C₂ à C₂₀ substitué ou non substitué, ou hétéroaryle à 6 chaînons substitué ou non substitué.

3. Complexe métallique de formule (I) selon la revendication 1 ou 2, dans lequel R¹ et R² sont identiques.

4. Complexe métallique de formule (I) selon l'une quelconque des revendications 1 à 3, dans lequel R¹ et/ou R² sont choisis parmi l'un des groupes D1 à D70 suivants (en tenant compte des conditions ci-dessus pour L) : 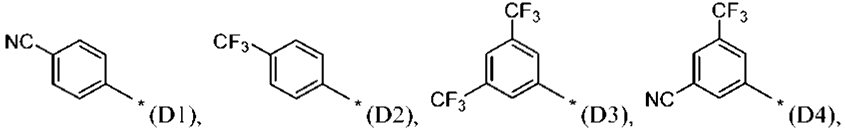 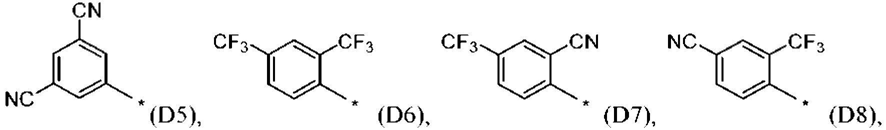 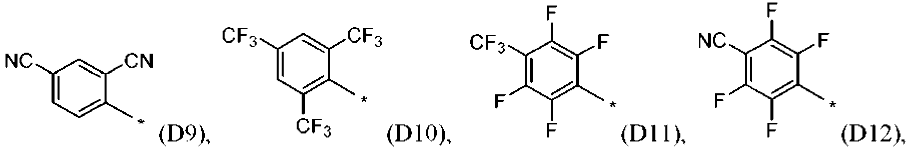 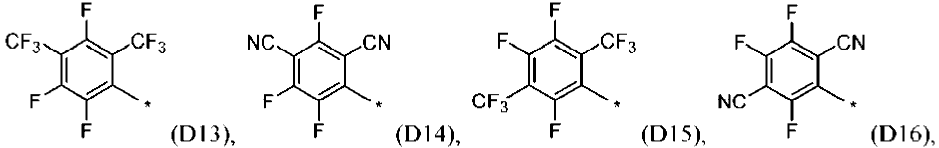 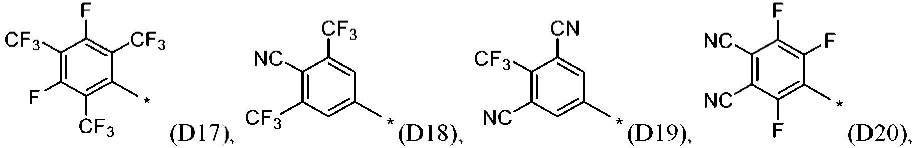 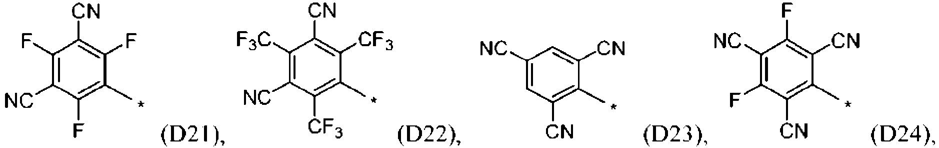 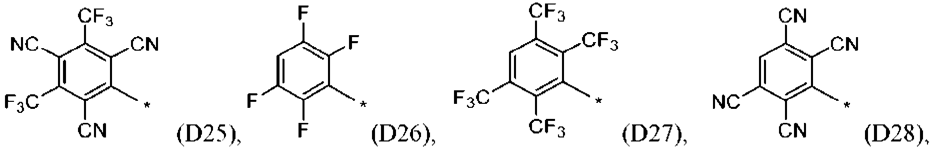 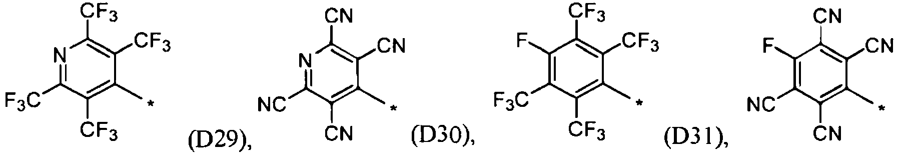 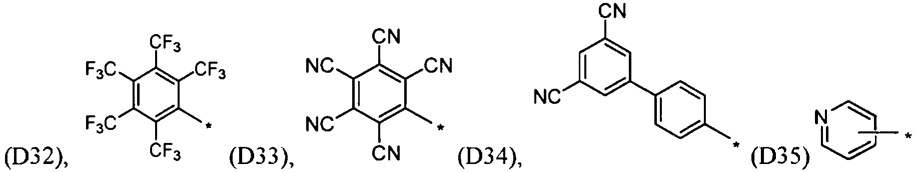 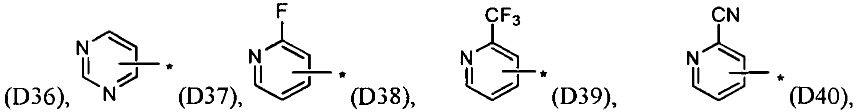 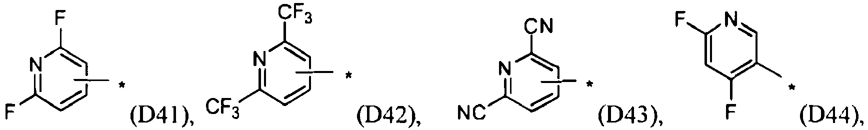 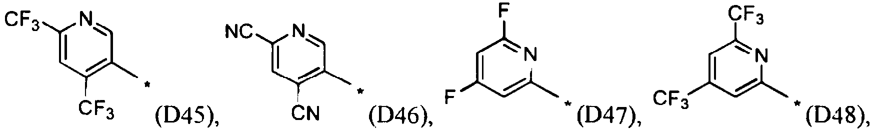 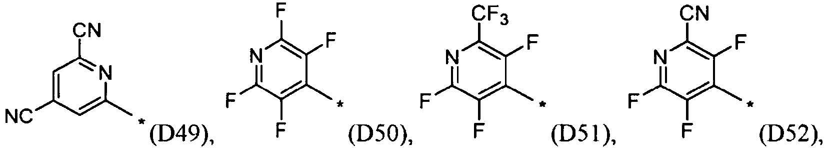 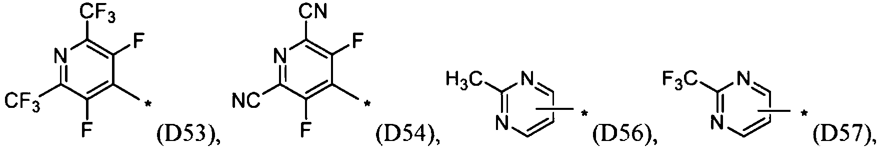 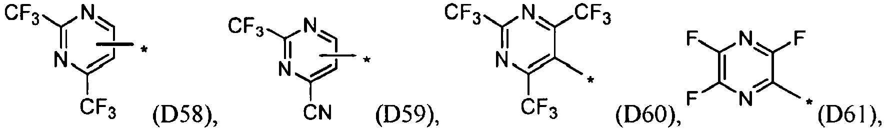 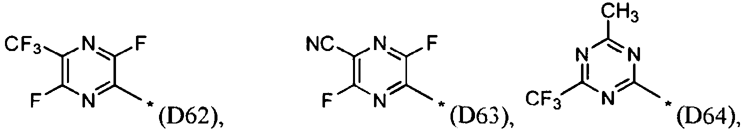 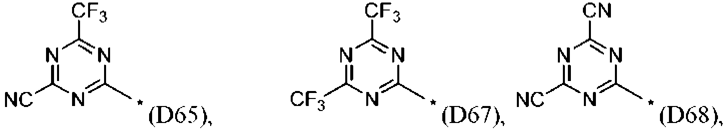 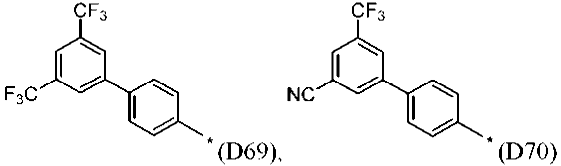 « * » indiquant la position de liaison.

5. Complexe métallique de formule (I) selon l'une quelconque des revendications 1 à 4, dans lequel le ligand L est choisi parmi l'un des groupes L1 à L54 suivants :

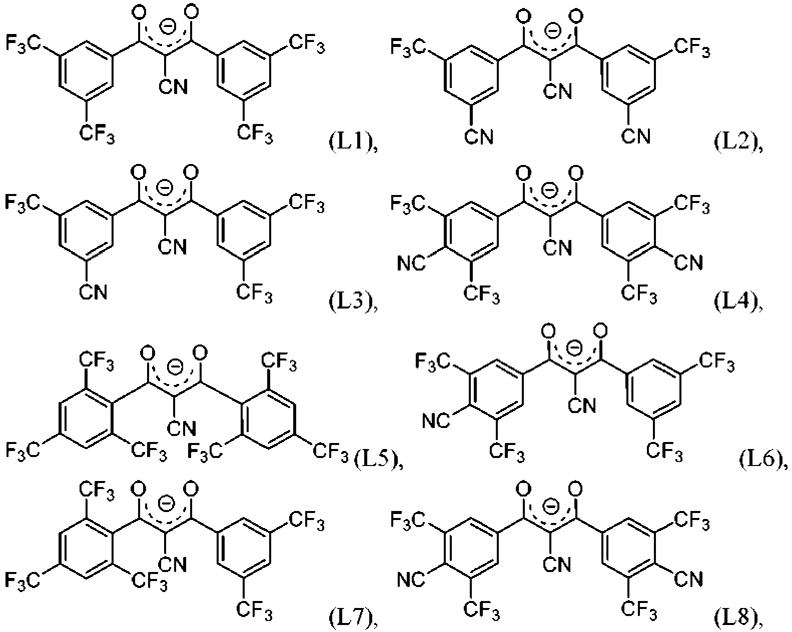

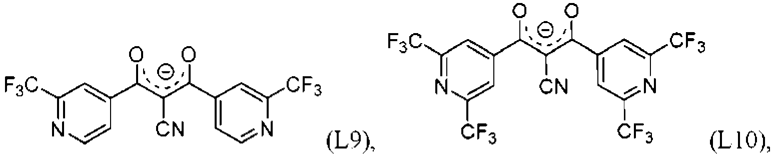

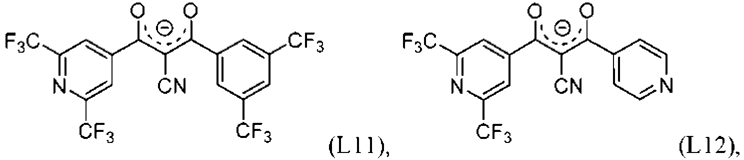

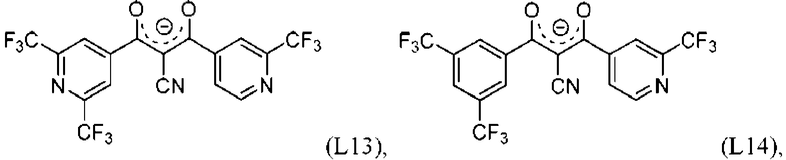

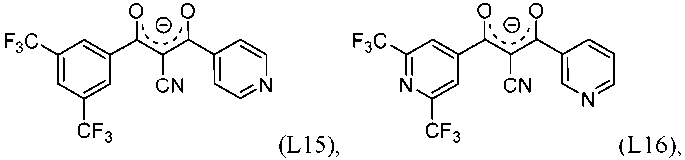

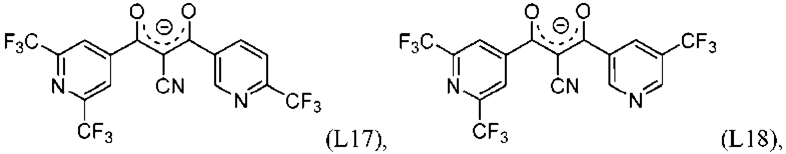

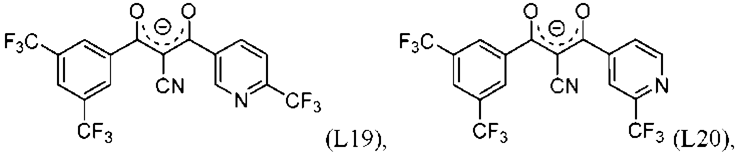

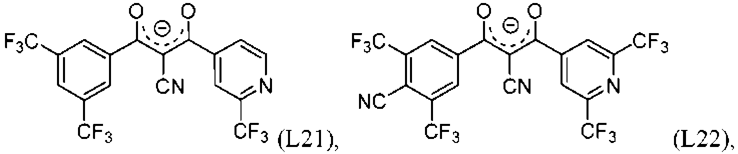

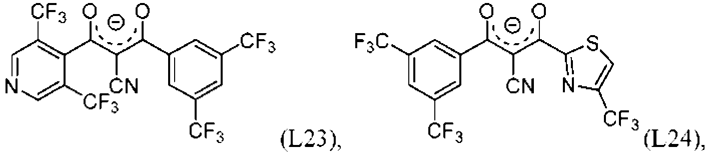

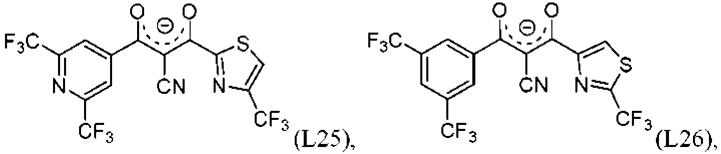

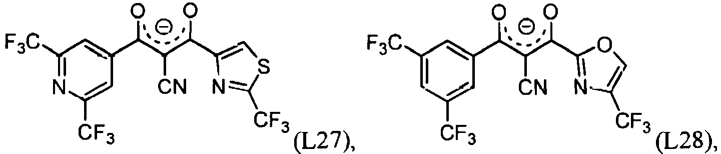

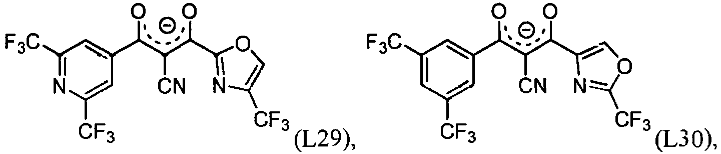

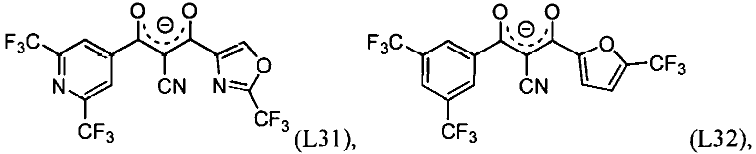

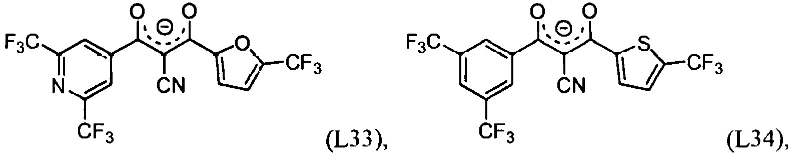

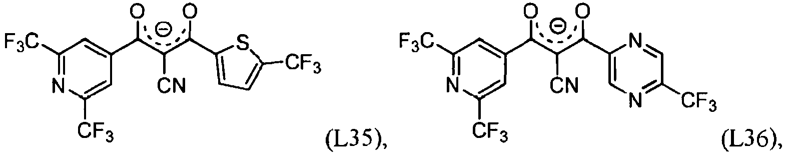

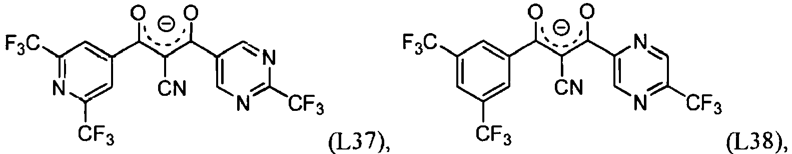

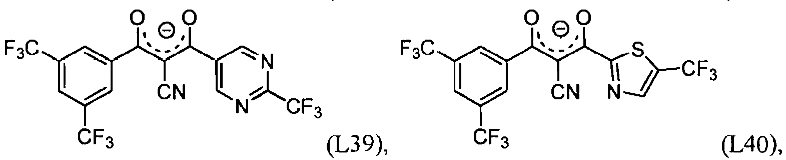

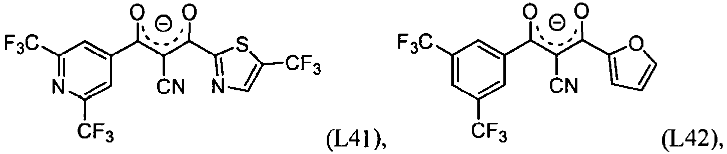

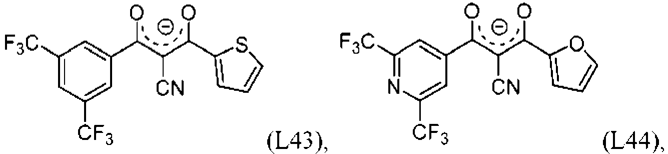

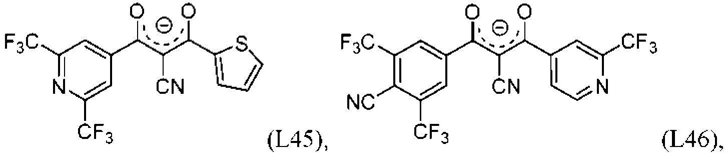

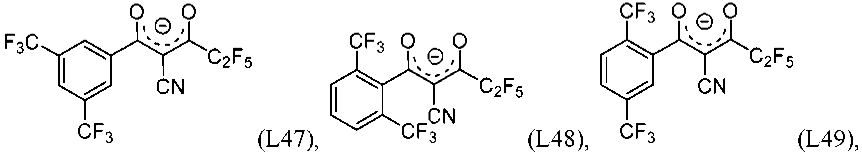

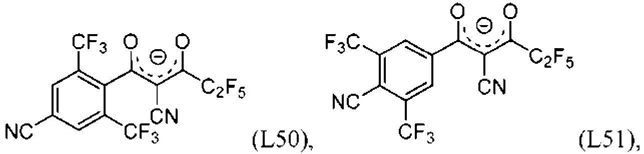

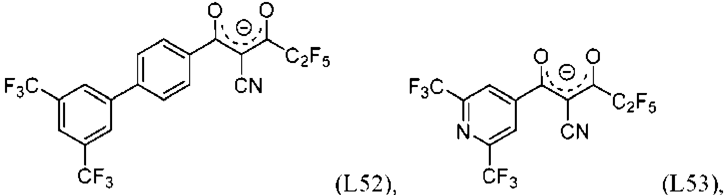

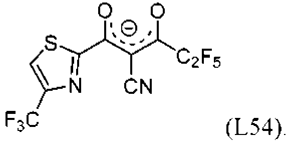

6. Couche de semi-conducteur organique, la couche de semi-conducteur organique comprenant un complexe métallique de formule (I) selon l'une quelconque des revendications 1 à 5 précédentes.

7. Dispositif électronique organique comprenant une couche d'anode, une couche de cathode et au moins une couche de semi-conducteur organique, l'au moins une couche de semi-conducteur organique étant disposée entre la couche d'anode et la couche de cathode, et l'au moins une couche de semi-conducteur organique étant la couche de semi-conducteur organique selon la revendication 6.

8. Dispositif électronique organique selon la revendication 7, la couche d'anode comprenant au moins une première sous-couche d'anode et une seconde sous-couche d'anode.

9. Dispositif électronique organique selon la revendication 7 ou 8, dans lequel le dispositif électronique organique comprend en outre au moins une couche photoactive, l'au moins une couche photoactive étant disposée entre la couche d'anode et la couche de cathode ; de préférence au moins une des au moins une couche de semi-conducteur organique étant disposée entre la couche d'anode et l'au moins une couche photoactive.

10. Dispositif électronique organique selon l'une quelconque des revendications 7 à 9 précédentes, la couche photoactive étant une couche émettrice de lumière.

11. Dispositif électronique organique selon l'une quelconque des revendications 7 à 10 précédentes, le dispositif électronique organique étant un dispositif électroluminescent, une diode émettrice de lumière organique (OLED), un dispositif émetteur de lumière, un transistor à couches minces, une batterie, un dispositif d'affichage ou une cellule photovoltaïque organique (OPV) .

12. Dispositif d'affichage comprenant un dispositif électronique organique selon l'une quelconque des revendications 7 à 11 précédentes.

13. Composé de formule (IIa) 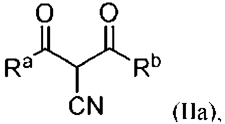
R^{a} étant choisi parmi alkyle en C₂ à C₁₂ substitué, aryle en C₆ à C₁₉ substitué, hétéroaryle en C₂ à C₂₀ substitué ou non substitué, hétéroaryle à 6 chaînons substitué ou non substitué ;
R^{b} étant choisi parmi les formules D38 à D68 telles que définies dans la revendication 4 ;
au moins un des substituants de l'alkyle en C₁ à C₁₂ substitué, l'aryle en C₆ à C₁₉ substitué, l'hétéroaryle en C₂ à C₂₀ substitué, ou l'hétéroaryle à 6 chaînons substitué étant indépendamment choisis parmi halogène, Cl, F, CN, alkyle en C₁ à C₈ partiellement fluoré ou perfluoré, alcoxy en C₁ à C₈ partiellement fluoré ou perfluoré ;
le composé comprenant au moins deux groupes CF₃ et/ou au moins un atome de N.

14. Composé selon la revendication 13, dans lequel R^{a} est choisi parmi alkyle en C₂ à C₁₂ substitué et aryle en C₆ à C₁₉ substitué et R^{b} est choisi parmi les formules D39, D42, D45, D48, D56, D57, D58, D67.
